# EUROPEAN PATENT SPECIFICATION

(11) **EP 3 086 761 B1**
(45) Date of publication and mention of the grant of the patent: **16.05.2018**
(21) Application number: 14819010.1
(22) Date of filing: 22.12.2014
(51) Int. Cl.: A61K 8/34, A61Q 1/04, A61K 8/73, A61K 8/891

(54) **AQUEOUS COSMETIC COMPOSITION COMPRISING ALKYLCELLULOSE, NON-VOLATILE OILS, A HYDROPHILIC GELLING POLYMER AND A POLYOL**
WÄSSRIGE KOSMETISCHE ZUSAMMENSETZUNG MIT ALKYLCELLULOSE, NICHT-FLÜCHTIGEN ÖLEN, EINEM HYDROPHILEN GELIERUNGSPOLYMER UND EINEM POLYOL
COMPOSITION COSMÉTIQUE AQUEUSE COMPRENANT UNE ALKYLCELLULOSE, DES HUILES NON VOLATILES, UN POLYMÈRE GÉLIFIANT HYDROPHILE ET UN POLYOL

(30) Priority: 23.12.2013 FR 1363420
(43) Date of publication of application: 02.11.2016
(73) Proprietor: L'OREAL, 75008 Paris (FR)
(72) Inventor: DURTSCHI, Aurore, F-91430 Vauhallan (FR); GUILLARD, Sylvie, F-77173 Chevry Cossigny (FR); AUGUSTE, Frédéric, 94550 Chevilly-Larue (FR); ARDITTY, Stéphane, 94550 Chevilly-la-Rue (FR)
(74) Representative: Wattremez, Catherine
(86) International application number: PCT/EP2014/079066
(87) International publication number: WO 2015/097181

(56) References cited:
- FR-A1- 2 889 955
- KR-A- 20130 005 565
- MINTEL: "BUTTER SHINE LIPSTICK", GNPD,, 5 September 2011 (2011-09-05), XP002658180,
- "Creamy Lip Color - Bobbi Brown", GNPD; MINTEL, 2011, XP002686248,

## Description

The present invention is directed towards proposing cosmetic compositions intended in particular for making up and/or caring for keratin materials. These compositions are in the form of an oil-in-water emulsion and comprise alkylcellulose, at least one polyol, in particular glycerol, and a polymer of natural origin as gelling agent, in combination with defined oils. These compositions are capable of affording a deposit, especially a makeup deposit, which has good cosmetic properties, in particular in terms of persistence, gloss, comfort and absence of tack.

In general, cosmetic compositions need to afford an aesthetic effect when applied to the skin and/or the lips, and to maintain this aesthetic effect over time.

In point of fact, the production of an aesthetic effect, after applying a cosmetic composition, results from a combination of cosmetic properties intrinsic to the composition which are expressed in terms of performance qualities such as the comfort on application and on wearing (especially being non-tacky or sparingly tacky), the uniformity, the transfer resistance and/or the persistence of the deposit over time and/or the gloss of the deposit.

In particular, producing a composition that is homogeneous and stable over time and also improving the persistence and/or the transfer resistance over time of cosmetic products, once they have been applied to the skin or the lips, is an ongoing concern for formulators. These objectives are particularly true in the field of makeup and in particular in the field of lipsticks, in stick form or in gloss form for the lips, and other skincare and/or lipcare products.

Ethylcellulose is known for its capacity, when it is dissolved in sufficient amount in cosmetic and/or therapeutic compositions, to enable the adherence of the resulting films.

Ethylcellulose, and alkylcelluloses in general (with an alkyl group comprising from 2 to 6 carbon atoms), unfortunately have limited solubility in the majority of the solvents commonly used in cosmetic and/or dermatological formulations. In general, monoalcohols containing from 2 to 8 carbon atoms, such as ethanol, butanol, methanol or isopropanol, are preferred for dissolving sufficient amounts of ethylcellulose in cosmetic or pharmaceutical compositions. Evaporation of the C₂-C₈ monoalcohols leads, after application of the corresponding cosmetic composition to the skin or the lips, firstly to concentration of the deposit and secondly to the formation of a coat on the surface of the skin or the lips that has good staying power. For example, document WO 96/36310 proposes cosmetic compositions especially comprising ethylcellulose dissolved in ethyl alcohol (SDA 38B-190 or SDA 40B-190 solvents).

However, these volatile monoalcohols have the drawback of being potentially irritant to the skin and/or the lips, and consequently may prove to be detrimental in the case of repeated use on the skin. However, when the presence of these compounds, especially such as ethanol, is dispensed with, this may prove to be detrimental in terms of the comfort and the tacky feel of the resulting deposit.

Cosmetic compositions in the form of an emulsion comprising water, non-volatile silicone-based and/or hydrocarbon-based oils and ethylcellulose have recently been proposed in patent application FR 2 964 868. Such compositions have the advantages of being homogeneous and stable over time (for example, no exudation or phase separation is observed, especially after 1 month at room temperature). They are easy to apply to the skin and/or the lips and afford a sensation of freshness that is unusual for this type of composition, in particular when they are intended to be applied to the lips. Furthermore, the deposit obtained is fine, light and uniform, with good properties in terms of gloss, and absence or limitation of tack. MINTEL: "BUTTER SHINE LIPSTICK", XP002658180, discloses (INCI declaration) a cosmetic composition comprising water, ethylcellulose, HEC, octyldodecanol, pentaerythryl adipate/caprate/caprylate/heptanoate, oleic acid, mentha oil, triethoxycaprylylsilane and ethylhexylglycerine. However, it is desirable to further increase the intensity of the coloration of the deposit obtained, without it being necessary to apply several layers of composition. It has, however, not been possible hitherto to increase the content of pigments without losing some of the advantages of these compositions, especially their stability, or the absence of tack of the deposit.

Furthermore, it is desired to improve the texture of these compositions and especially to make them more creamy, in order to increase the comfort of the deposit, without degrading the advantageous properties thereof detailed above.

The object of the present invention is, precisely, to satisfy these needs.

Thus, according to a first of its aspects, a subject of the present invention is a cosmetic composition in the form of an oil-in-water emulsion comprising:
(A) at least 5% by weight of water relative to the total weight of the composition;
(B) at least 4% by weight of alkylcellulose, the alkyl residue of which comprises between 2 and 6 carbon atoms;
(C) at least one hydrophilic gelling agent chosen from optionally modified polymers of natural origin;
(D) at least one saturated or non-saturated, linear or branched C₂-C₈ and preferably C₃-C₆ polyol, comprising from 2 to 6 hydroxyl groups, and preferably glycerol, propylene glycol, 1,3-butylene glycol, dipropylene glycol or diglycerol, and a mixture thereof;
(E) at least one hydrocarbon-based non-volatile first oil chosen from:
   - C₁₀-C₂₆ alcohols, preferably monoalcohols;
   - optionally hydroxylated monoesters, diesters or triesters of a C₂-C₈ monocarboxylic or polycarboxylic acid and of a C₂-C₈ alcohol, which are optionally hydroxylated;
   - esters of a C₂-C₈ polyol and of one or more C₂-C₈ carboxylic acids; and
(F) at least one non-volatile second oil chosen from silicone oils and/or fluoro oils or hydrocarbon-based oils other than the first oils (E).

As emerges from the example presented below, the inventors have discovered, surprisingly, that an oil-in-water emulsion comprising a combination of ethylcellulose with a particular oily system, combined with glycerol and with a hydrophilic gelling agent chosen from modified or unmodified polymers of natural origin, makes it possible to obtain a uniform, stable composition whose texture is much more creamy and which affords access to deposits whose coloration intensity is visibly improved, with satisfactory gloss.

It has also been found that the composition is still just as easy to apply and gives a makeup result with precise contours of the lips. In addition, the deposit obtained remains fine and uniform, still with this original impression of freshness on application. The deposit shows good persistence and, surprisingly, affords a comfort sensation that is prolonged over time.

The composition according to the invention is more particularly in liquid form.

For the purposes of the present invention, the term "liquid" (or "fluid") is intended to denote a composition that is capable of flowing under its own weight, at room temperature (at 20°C) and at atmospheric pressure (760 mmHg), as opposed to a solid composition.

In particular, the term "liquid" means a fluid texture, i.e. which may especially be in creamy or pasty form.

### Protocol for measuring the viscosity:

The viscosity measurement is generally performed at 20°C, using a Rheomat RM180 viscometer equipped with a No. 2, 3 or 4 spindle, the measurement being performed after 10 minutes of rotation of the spindle in the composition (after which time stabilization of the viscosity and of the spin speed of the spindle are observed), at a shear rate of 200 rpm.

Preferably, the composition according to the invention may have at 25°C a viscosity of between 0.2 and 25 Pa.s and preferably between 1 and 20 Pa.s.

Preferably, the viscosity at 25°C of a composition according to the invention may be between 2 and 17 Pa.s.

In particular, the viscosity at 25°C of a composition according to the invention may be between 0.2 Pa.s (spindle 2) and 25 Pa.s (spindle 4), preferably between 1 Pa.s (spindle 2) and 17 Pa.s (spindle 4) and better still between 1 Pa.s (spindle 2) and 12 Pa.s (spindle 4).

As indicated previously, the composition according to the invention is in the form of an oil-in-water emulsion.

Preferably, the cosmetic composition according to the invention is a lipstick, especially a liquid lipstick, or a lip gloss.

The compositions according to the invention may especially be in gloss form, intended for making up and/or caring for keratin materials, in particular the skin and/or the lips, and better still the lips.

According to another of its aspects, a subject of the present patent application is a cosmetic process for making up and/or caring for keratin materials, in particular the lips and/or the skin, better still the lips, comprising at least one step that consists in applying to the said keratin materials at least one composition as defined previously.

### ETHYLCELLULOSE

A composition according to the invention comprises at least alkylcellulose, the alkyl residue of which comprises between 2 and 6 carbon atoms and preferably between 2 and 3 carbon atoms, and better still a composition according to the invention comprises ethylcellulose.

According to a particularly preferred embodiment, the alkylcellulose (the alkyl residue of which preferably comprises between 2 and 6 carbon atoms, preferentially ethylcellulose) may be present in a composition according to the invention in a (solids) content ranging from 1% to 60% by weight.

Particularly preferably, the composition according to the invention may comprise from 4% to 50% by dry weight of alkylcellulose (the alkyl residue of which preferably comprises between 2 and 6 carbon atoms, preferably ethylcellulose), more preferably from 5% to 30% by weight and even more preferably from 5% to 20% by weight, relative to the total weight of the said composition.

The alkylcellulose is a cellulose alkyl ether comprising a chain formed from β-anhydroglucose units linked together via acetal bonds. Each anhydroglucose unit contains three replaceable hydroxyl groups, all or some of these hydroxyl groups being able to react according to the following reaction:

RONa + C₂H₅Cl → ROC₂H₅ + NaCl,

in which R represents a cellulose radical.

Advantageously, the alkylcellulose may be chosen from ethylcellulose and propylcellulose.

According to a particularly preferred embodiment, the alkylcellulose may be ethylcellulose.

It is a cellulose ethyl ether.

Total substitution of the three hydroxyl groups would lead for each anhydroglucose unit to a degree of substitution of 3, in other words to a content of alkoxy groups of 54.88%.

The ethylcellulose polymers used in a cosmetic composition according to the invention are preferentially polymers with a degree of substitution with ethoxy groups ranging from 2.5 to 2.6 per anhydroglucose unit, in other words comprising a content of ethoxy groups ranging from 44% to 50%.

According to a preferred mode, the alkylcellulose (preferably ethylcellulose) may be used in a composition of the invention in the form of particles dispersed in an aqueous phase, like a dispersion of latex or pseudolatex type. The techniques for preparing these latex dispersions are well known to those skilled in the art.

The product sold by the company FMC Biopolymer under the name Aquacoat ECD-30, which consists of a dispersion of ethylcellulose in a proportion of about 26.2% by weight in water and stabilized with sodium lauryl sulfate and cetyl alcohol, is most particularly suitable for use as an aqueous dispersion of ethylcellulose.

According to a particular embodiment, the aqueous dispersion of ethylcellulose, in particular the product Aquacoat ECD, may be used in a proportion of from 10% to 90% by weight, in particular from 15% to 60% by weight and preferably from 20% to 50% by weight of ethylcellulose dispersion, relative to the total weight of the composition.

### PHYSIOLOGICALLY ACCEPTABLE MEDIUM

Besides the compounds indicated previously, the composition according to the invention comprises a physiologically acceptable medium.

The term "physiologically acceptable medium" is intended to denote a medium that is particularly suitable for applying a composition of the invention to keratin materials, in particular the skin and/or the lips, for instance water, oils other than those indicated above or organic solvents commonly used in cosmetic compositions.

The physiologically acceptable medium (acceptable tolerance, toxicology and feel) is generally adapted to the nature of the support onto which the composition is to be applied, and also to the form in which the composition is to be conditioned.

### NON-VOLATILE FIRST OILS (E)

As indicated previously, the composition according to the invention comprises at least one particular hydrocarbon-based non-volatile oil.

The term "non-volatile" is intended to mean an oil of which the vapour pressure at 25°C and atmospheric pressure is non-zero and is less than 0.02 mmHg (2.66 Pa) and better still less than 10⁻³ mmHg (0.13 Pa).

This oil is chosen from:

### - C₁₀-C₂₆ alcohols, preferably monoalcohols.

More particularly, the C₁₀-C₂₆ alcohols are saturated or unsaturated, and branched or unbranched, and comprise from 10 to 26 carbon atoms.

Preferably, the C₁₀-C₂₆ alcohols are fatty alcohols, which are preferably branched when they comprise at least 16 carbon atoms.

As examples of fatty alcohols that may be used according to the invention, mention may be made of linear or branched fatty alcohols, of synthetic origin or alternatively of natural origin, for example alcohols derived from plant material (coconut, palm kernel, palm, etc.) or animal material (tallow, etc.).

Needless to say, other long-chain alcohols may also be used, for instance ether alcohols or alternatively "Guerbet" alcohols.

Finally, use may also be made of certain more or less long fractions of alcohols of natural origin, for instance coconut (C₁₂ to C₁₆) or tallow (C₁₆ to C₁₈) or compounds of diol or cholesterol type.

Use is preferably made of a fatty alcohol comprising from 10 to 24 carbon atoms and more preferentially from 12 to 22 carbon atoms.

As particular examples of fatty alcohols that may preferably be used, mention may be made especially of lauryl alcohol, isostearyl alcohol, oleyl alcohol, 2-butyloctanol, 2-undecylpentadecanol, 2-hexyldecyl alcohol, isocetyl alcohol and octyldodecanol, and mixtures thereof.

According to one advantageous embodiment of the invention, the alcohol is chosen from octyldodecanol;
- **optionally hydroxylated monoesters, diesters or triesters of a C₂-C₈ monocarboxylic or polycarboxylic acid and of a C₂-C₈ alcohol.**
   In particular:
   * optionally hydroxylated monoesters of a C₂-C₈ carboxylic acid and of a C₂-C₈ alcohol,
   * optionally hydroxylated diesters of a C₂-C₈ dicarboxylic acid and of a C₂-C₈ alcohol, such as diisopropyl adipate, 2-diethylhexyl adipate, dibutyl adipate, diisostearyl adipate or 2-diethylhexyl succinate,
   * optionally hydroxylated triesters of a C₂-C₈ tricarboxylic acid and of a C₂-C₈ alcohol, such as citric acid esters, such as trioctyl citrate, triethyl citrate, acetyl tributyl citrate, tributyl citrate or acetyl tributyl citrate;
- **esters of a C₂-C₈ polyol and of one or more C₂-C₈ carboxylic acids,** such as glycol diesters of monoacids, such as neopentyl glycol diheptanoate, or glycol triesters of monoacids, such as triacetin.

Preferably, the first oil is chosen from C₁₀-C₂₆ alcohols, more particularly monoalcohols and preferably octyldodecanol.

According to an advantageous embodiment of the invention, the content of first non-volatile hydrocarbon-based oil(s) (E) ranges from 5% to 75% by weight, in particular from 10% to 50% by weight and preferably from 20% to 45% by weight, relative to the total weight of the composition.

### NON-VOLATILE SECOND OILS (F)

The composition according to the invention comprises at least one non-volatile second oil chosen from non-volatile silicone oils and/or volatile hydrocarbon-based oils other than the abovementioned oils (E).

The non-volatile oil(s) are more particularly chosen from non-volatile silicone oils, which may or may not be phenylated; non-volatile fluoro oils; polar or apolar non-volatile hydrocarbon-based oils other than the abovementioned non-volatile oils (E); or mixtures thereof.

### Silicone oils

The term "silicone oil" means an oil containing at least one silicon atom, and in particular containing Si-O groups.

### Non-volatile non-phenylated silicone oils

The term "non-phenylated silicone oil" denotes a silicone oil not bearing any phenyl substituents.

Representative examples of these non-volatile non-phenylated silicone oils which may be mentioned include polydimethylsiloxanes; alkyl dimethicones; vinylmethyl methicones; and also silicones modified with aliphatic groups and/or with functional groups such as hydroxyl, thiol and/or amine groups.

It should be noted that "dimethicone" (INCI name) corresponds to a polydimethylsiloxane (chemical name).

The non-volatile non-phenylated silicone oil is preferably chosen from non-volatile dimethicone oils.

In particular, these oils can be chosen from the following non-volatile oils:
- polydimethylsiloxanes (PDMSs),
- PDMSs comprising aliphatic groups, in particular alkyl or alkoxy groups, which are pendent and/or at the end of the silicone chain, these groups each comprising from 2 to 24 carbon atoms. By way of example, mention may be made of the cetyl dimethicone sold under the commercial reference Abil Wax 9801 from Evonik Goldschmidt,
- PDMSs comprising aliphatic groups, or functional groups such as hydroxyl, thiol and/or amine groups,
- polyalkylmethylsiloxanes substituted with functional groups such as hydroxyl, thiol and/or amine groups,
- polysiloxanes modified with fatty acids, fatty alcohols or polyoxyalkylenes, and mixtures thereof.

Preferably, these non-volatile non-phenylated silicone oils are chosen from polydimethylsiloxanes; alkyl dimethicones and also PDMSs comprising aliphatic groups, in particular C₂-C₂₄ alkyl groups, and/or functional groups such as hydroxyl, thiol and/or amine groups.

The non-phenylated silicone oil may be chosen in particular from silicones of formula (I): 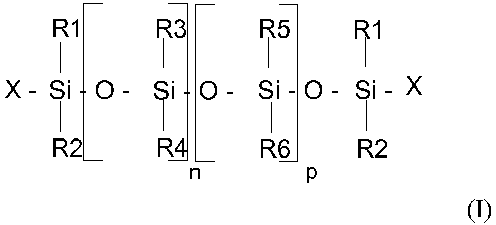 in which:
R₁, R₂, R₅ and R₆ are, together or separately, an alkyl radical containing from 1 to 6 carbon atoms,
R₃ and R₄ are, together or separately, an alkyl radical containing from 1 to 6 carbon atoms, a vinyl radical, an amine radical or a hydroxyl radical,
X is an alkyl radical containing from 1 to 6 carbon atoms, a hydroxyl radical or an amine radical,
n and p are integers chosen so as to have a fluid compound, in particular of which the viscosity at 25°C is between 9 centistokes (cSt) (9 x 10⁻⁶ m²/s) and 800 000 cSt.

As non-volatile non-phenylated silicone oils which can be used according to the invention, mention may be made of those for which:
- the substituents R₁ to R₆ and X represent a methyl group, and p and n are such that the viscosity is 500 000 cSt, for example the product sold under the name SE30 by the company General Electric, the product sold under the name AK 500000 by the company Wacker, the product sold under the name Mirasil DM 500 000 by the company Bluestar, and the product sold under the name Dow Corning 200 Fluid 500 000 cSt by the company Dow Corning,
- the substituents R₁ to R₆ and X represent a methyl group, and p and n are such that the viscosity is 60 000 cSt, for example the product sold under the name Dow Corning 200 Fluid 60 000 CS by the company Dow Corning, and the product sold under the name Wacker Belsil DM 60 000 by the company Wacker,
- the substituents R₁ to R₆ and X represent a methyl group, and p and n are such that the viscosity is 100 cSt or 350 cSt, for example the products sold respectively under the names Belsil DM100 and Dow Corning 200 Fluid 350 CS by the company Dow Corning,
- the substituents R₁ to R₆ represent a methyl group, the group X represents a hydroxyl group, and n and p are such that the viscosity is 700 cSt, for example the product sold under the name Baysilone Fluid T0.7 by the company Momentive.

### Non-volatile phenyl silicone oils

The expression "phenyl silicone oil" denotes a silicone oil bearing at least one phenyl substituent.

These non-volatile phenyl silicone oils may be chosen from those also bearing at least one dimethicone fragment, or from those not bearing one.

The term "dimethicone fragment" denotes a divalent siloxane group in which the silicon atom bears two methyl radicals, this group not being located at the ends of the molecule. It may be represented by the following formula: -(Si(CH₃)₂-O)-.

The non-volatile phenyl silicone oil may thus be chosen from:
**a)** phenyl silicone oils optionally bearing a dimethicone fragment corresponding to formula (I) below: 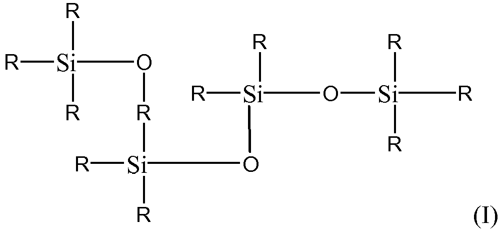 in which the groups R, which are monovalent or divalent, represent, independently of each other, a methyl or a phenyl, with the proviso that at least one group R represents a phenyl.
   Preferably, in this formula, the phenyl silicone oil comprises at least three phenyl groups, for example at least four, at least five or at least six.
**b)** phenyl silicone oils optionally bearing a dimethicone fragment corresponding to formula (II) below: 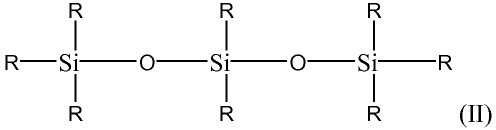 in which the groups R represent, independently of each other, a methyl or a phenyl, with the proviso that at least one group R represents a phenyl.

Preferably, in this formula, the compound of formula (II) comprises at least three, for example at least four or at least five, phenyl groups.

Mixtures of different phenyl silicone compounds described previously may be used.

Examples that may be mentioned include mixtures of triphenyl-, tetraphenyl- or pentaphenyl-organopolysiloxanes.

Among the compounds of formula (II), mention may be made more particularly of phenyl silicone oils not bearing a dimethicone fragment, corresponding to formula (II) in which at least 4 or at least 5 radicals R represent a phenyl radical, the remaining radicals representing methyls.

Such non-volatile phenyl silicone oils are preferably trimethylpentaphenyltrisiloxane or tetramethyltetraphenyltrisiloxane. They are in particular sold by Dow Corning under the reference PH-1555 HRI or Dow Corning 555 Cosmetic Fluid (chemical name: 1,3,5-trimethyl-1,1,3,5,5-pentaphenyltrisiloxane; INCI name: trimethylpentaphenyltrisiloxane), or the tetramethyltetraphenyltrisiloxane sold under the reference Dow Corning 554 Cosmetic Fluid by Dow Corning can also be used.

They correspond especially to the following formulae (III), (III'): 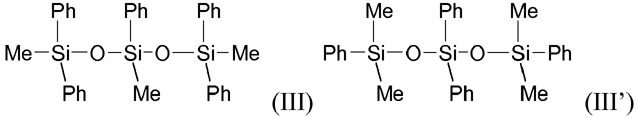 in which Me represents methyl, and Ph represents phenyl.
**c)** phenyl silicone oils bearing at least one dimethicone fragment corresponding to formula (IV) below: 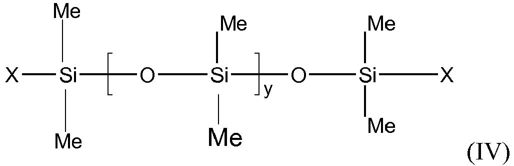 in which Me represents methyl, y is between 1 and 1000 and X represents -CH₂-CH(CH₃)(Ph).
**d)** phenyl silicone oils corresponding to formula (V) below, and mixtures thereof: 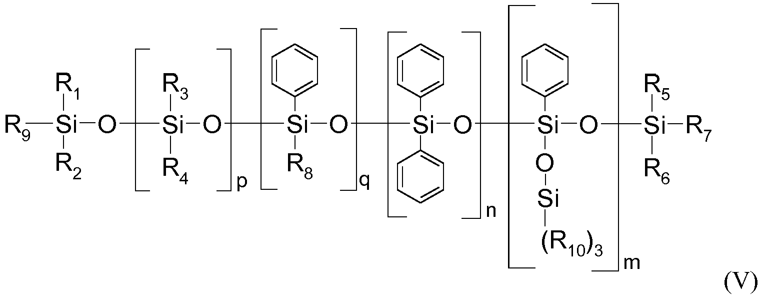 in which:
   - R₁ to R₁₀, independently of each other, are saturated or unsaturated, linear, cyclic or branched C₁-C₃₀ hydrocarbon-based radicals,
   - m, n, p and q are, independently of each other, integers between 0 and 900, with the proviso that the sum m+n+q is other than 0.

Preferably, the sum m+n+q is between 1 and 100. Advantageously, the sum m+n+p+q is between 1 and 900 and preferably between 1 and 800.

Preferably, q is equal to 0.

More particularly, R₁ to R₁₀, independently of each other, represent a saturated or unsaturated, preferably saturated, linear or branched C₁-C₃₀ hydrocarbon-based radical, and in particular a preferably saturated, C₁-C₂₀, in particular C₁-C₁₈, hydrocarbon-based radical, or a monocyclic or polycyclic C₆-C₁₄, and in particular C₁₀-C₁₃, aryl radical, or an aralkyl radical, the alkyl part of which is preferably C₁-C₃ alkyl.

Preferably, R₁ to R₁₀ may each represent a methyl, ethyl, propyl, butyl, isopropyl, decyl, dodecyl or octadecyl radical, or alternatively a phenyl, tolyl, benzyl or phenethyl radical. R₁ to R₁₀ may in particular be identical, and in addition may be a methyl radical.

According to a first more particular embodiment of formula (V), mention may be made of:
**i)** phenyl silicone oils optionally bearing at least one dimethicone fragment corresponding to formula (VI) below, and mixtures thereof: 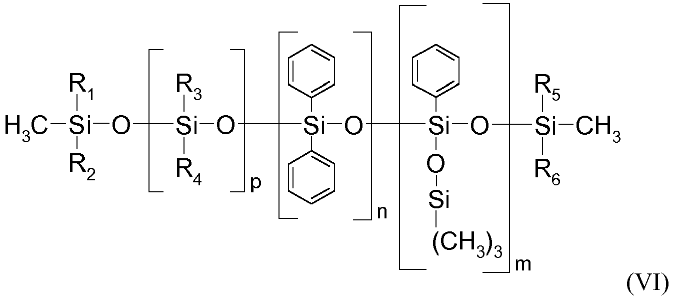 in which:
   - R₁ to R₆, independently of each other, are saturated or unsaturated, linear, cyclic or branched C₁-C₃₀ hydrocarbon-based radicals, a preferably C₆-C₁₄ aryl radical or an aralkyl radical, the alkyl part of which is C₁-C₃ alkyl,
   - m, n and p are, independently of each other, integers between 0 and 100, with the proviso that the sum n+m is between 1 and 100.

Preferably, R₁ to R₆, independently of each other, represent a C₁-C₂₀, in particular C₁-C₁₈, hydrocarbon-based, preferably alkyl, radical, or a C₆-C₁₄ aryl radical which is monocyclic (preferably C₆) or polycyclic and in particular C₁₀-C₁₃, or an aralkyl radical (preferably the aryl part is C₆ aryl; the alkyl part is C₁-C₃ alkyl).

Preferably, R₁ to R₆ may each represent a methyl, ethyl, propyl, butyl, isopropyl, decyl, dodecyl or octadecyl radical, or alternatively a phenyl, tolyl, benzyl or phenethyl radical.

R₁ to R₆ may in particular be identical, and in addition may be a methyl radical. Preferably, m = 1 or 2 or 3, and/or n = 0 and/or p = 0 or 1 can be applied, in formula (VI).

According to one particular embodiment, the non-volatile phenyl silicone oil is chosen from phenyl silicone oils bearing at least one dimethicone fragment.

Preferably, such oils correspond to compounds of formula (VI) in which:
**A)** m=0 and n and p are, independently of each other, integers between 1 and 100.
   Preferably, R₁ to R₆ are methyl radicals.
   According to this embodiment, the silicone oil is preferably chosen from a diphenyl dimethicone such as KF-54 from Shin Etsu (400 cSt), KF54HV from Shin Etsu (5000 cSt), KF-50-300CS from Shin Etsu (300 cSt), KF-53 from Shin Etsu (175 cSt) or KF-50-100CS from Shin Etsu (100 cSt).
**B)** p is between 1 and 100, the sum n+m is between 1 and 100, and n=0.

These phenyl silicone oils optionally bearing at least one dimethicone fragment correspond more particularly to formula (VII) below: 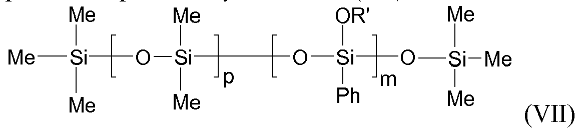 in which Me is methyl and Ph is phenyl, OR' represents a group
-OSiMe₃ and p is 0 or is between 1 and 1000, and m is between 1 and 1000. In particular, m and p are such that the compound (VII) is a non-volatile oil.

According to a first embodiment of non-volatile phenyl silicone bearing at least one dimethicone fragment, p is between 1 and 1000 and m is more particularly such that the compound (VII) is a non-volatile oil. Trimethylsiloxyphenyldimethicone, sold in particular under the reference Belsil PDM 1000 by the company Wacker, may, for example, be used.

According to a second embodiment of non-volatile phenyl silicone not bearing a dimethicone fragment, p is equal to 0 and m is between 1 and 1000, and in particular is such that the compound (VII) is a non-volatile oil.

Phenyltrimethylsiloxytrisiloxane, sold in particular under the reference Dow Corning 556 Cosmetic Grade Fluid (DC556), may, for example, be used.
**ii)** non-volatile phenyl silicone oils not bearing a dimethicone fragment corresponding to formula (VIII) below, and mixtures thereof: 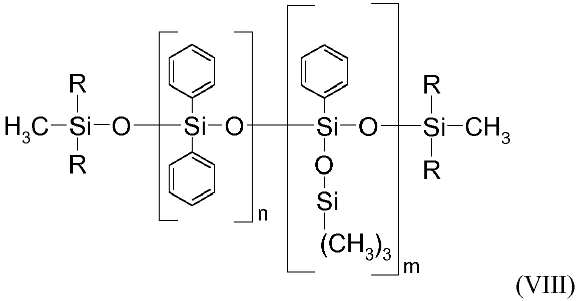 in which:
   - R, independently of each other, are saturated or unsaturated, linear, cyclic or branched C₁-C₃₀ hydrocarbon-based radicals, preferably R is a C₁-C₃₀ alkyl radical, a preferably C₆-C₁₄ aryl radical, or an aralkyl radical, the alkyl part of which is C₁-C₃ alkyl,
   - m and n are, independently of each other, integers between 0 and 100, with the proviso that the sum n+m is between 1 and 100.

Preferably, R, independently of each other, represent a saturated or unsaturated, preferably saturated, linear or branched C₁-C₃₀ hydrocarbon-based radical, and in particular a preferably saturated, C₁-C₂₀, in particular C₁-C₁₈ and more particularly C₄-C₁₀, hydrocarbon-based radical, a monocyclic or polycyclic C₆-C₁₄, and in particular C₁₀-C₁₃, aryl radical, or an aralkyl radical of which preferably the aryl part is C₆ aryl and the alkyl part is C₁-C₃ alkyl.

Preferably, the Rs may each represent a methyl, ethyl, propyl, butyl, isopropyl, decyl, dodecyl or octadecyl radical, or alternatively a phenyl, tolyl, benzyl or phenethyl radical.

The radicals R may in particular be identical, and in addition may be a methyl radical.

Preferably, m = 1 or 2 or 3, and/or n = 0 and/or p = 0 or 1 can be applied, in formula (VIII).

According to one preferred embodiment, n is an integer between 0 and 100 and m is an integer between 1 and 100, with the proviso that the sum n+m is between 1 and 100, in formula (VIII). Preferably, R is a methyl radical.

According to one embodiment, a phenyl silicone oil of formula (VIII) with a viscosity at 25°C of between 5 and 1500 mm²/s (i.e. 5 to 1500 cSt), and preferably with a viscosity of between 5 and 1000 mm²/s (i.e. 5 to 1000 cSt), may be used.

According to this embodiment, the non-volatile phenyl silicone oil is preferably chosen from phenyl trimethicones (when n=0) such as DC556 from Dow Corning (22.5 cSt), or else from diphenylsiloxyphenyl trimethicone oil (when m and n are between 1 and 100) such as KF56 A from Shin Etsu, or the Silbione 70663V30 oil from Rhône-Poulenc (28 cSt). The values in parentheses represent the viscosities at 25°C.
**e)** phenyl silicone oils optionally bearing at least one dimethicone fragment corresponding to the following formula, and mixtures thereof: 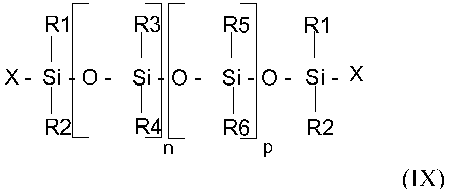 in which:
   R₁, R₂, R₅ and R₆, which may be identical or different, are an alkyl radical containing 1 to 6 carbon atoms,
   R₃ and R₄, which may be identical or different, are an alkyl radical containing from 1 to 6 carbon atoms or an aryl radical (preferably C₆-C₁₄), with the proviso that at least one of R₃ and R₄ is a phenyl radical,
   X is an alkyl radical containing from 1 to 6 carbon atoms, a hydroxyl radical or a vinyl radical,
   n and p being an integer greater than or equal to 1, chosen so as to give the oil a weight-average molecular weight of less than 200 000 g/mol, preferably less than 150 000 g/mol and more preferably less than 100 000 g/mol.
**f)** and a mixture thereof.

### Non-volatile fluoro oils

The term "fluoro oil" means an oil containing at least one fluorine atom.

As examples of fluoro oils, mention may be made of fluorosilicone oils, fluorinated polyethers, fluorosilicones in particular as described in document EP-A-847 752 and perfluoro compounds.

According to the invention, the term "perfluoro compounds" is intended to mean compounds in which all the hydrogen atoms have been replaced with fluorine atoms.

According to a preferred embodiment, the fluoro oil is chosen from perfluoro oils.

As examples of perfluoro oils, mention may be made of perfluorodecalins and perfluoroperhydrophenanthrenes.

According to one preferred embodiment, the fluoro oil is chosen from perfluoroperhydrophenanthrenes, and in particular the Fiflow® products sold by the company Créations Couleurs. In particular, use may be made of the fluoro oil of which the INCI name is Perfluoroperhydrophenanthrene, sold under the reference Fiflow 220 by the company F2 Chemicals.

### Polar non-volatile hydrocarbon-based oils

The term "hydrocarbon-based oil" is intended to mean an oil formed essentially from, or even constituted by, carbon and hydrogen atoms, and possibly oxygen and nitrogen atoms, and not containing any silicon or fluorine atoms.

It may contain alcohol, ester, ether, carboxylic acid, amine and/or amide groups.

Preferably, the hydrocarbon-based oil, in addition to being free of silicon and fluorine, is free of heteroatoms such as N, Si and P. The hydrocarbon-based oil is therefore different from a silicone oil and from a fluoro oil.

In the present case, the non-volatile hydrocarbon-based oil comprises at least one oxygen atom.

In particular, this non-volatile hydrocarbon-based oil, other than the non-volatile hydrocarbon-based oils (E), is chosen from ester oils containing at least 18 carbon atoms, in particular containing between 18 and 70 carbon atoms, the oils comprising at least one carbonate function, and mixtures thereof.

As regards the ester oils containing at least 18 carbon atoms, mention may be made of monoesters, diesters or triesters.

The ester oils may be hydroxylated or non-hydroxylated.

Thus, the non-volatile ester oil that is suitable for use in the invention may be chosen from:
*** monoesters comprising at least 18 carbon atoms,** in particular comprising between 18 and 40 carbon atoms in total, in particular the monoesters of formula R₁COOR₂ in which R₁ represents a saturated or unsaturated, linear or branched or aromatic fatty acid residue comprising from 4 to 40 carbon atoms and R₂ represents a hydrocarbon-based chain, which is in particular branched, containing from 4 to 40 carbon atoms, on condition that R₁ + R₂ ≥ 18, for instance Purcellin oil (cetostearyl octanoate), isononyl isononanoate, C₁₂ to C₁₅ alkyl benzoate, 2-ethylhexyl palmitate, octyldodecyl neopentanoate, 2-octyldodecyl stearate, 2-octyldodecyl erucate, isostearyl isostearate, 2-octyldodecyl benzoate, alcohol or polyalcohol octanoates, decanoates or ricinoleates, isopropyl myristate, isopropyl palmitate, butyl stearate, hexyl laurate, 2-ethylhexyl palmitate, 2-hexyldecyl laurate, 2-octyldecyl palmitate or 2-octyldodecylmyristate.

Preferably, they are esters of formula R₁COOR₂ in which R₁ represents a linear or branched fatty acid residue containing from 4 to 40 carbon atoms and R₂ represents a hydrocarbon-based chain that is in particular branched, containing from 4 to 40 carbon atoms, R₁ and R₂ being such that R₁ + R₂ ≥ 18.

Even more particularly, the ester comprises between 18 and 40 carbon atoms in total.

Preferred monoesters that may be mentioned include isononyl isononanoate, oleyl erucate and/or 2-octyldodecyl neopentanoate;
*** monoesters of a fatty acid comprising at least 18 carbon atoms,** and in particular containing from 18 to 22 carbon atoms, and of diols. They may especially be esters of lanolic acid, oleic acid, lauric acid or stearic acid, and of diols, for instance propylene glycol monoisostearate;
*** diesters comprising at least 18 carbon atoms,** especially comprising between 18 and 60 carbon atoms in total and in particular between 18 and 50 carbon atoms in total. Use may thus be made of diesters of a dicarboxylic acid and of monoalcohols, preferably such as diisostearyl malate, or glycol diesters of monocarboxylic acids, such as neopentyl glycol diheptanoate, propylene glycol dioctanoate, diethylene glycol diisononanoate or polyglyceryl-2 diisostearate (in particular such as the compound sold under the commercial reference Dermol DGDIS by the company Alzo);
*** hydroxylated monoesters and diesters comprising at least 18 carbon atoms,** preferably with a total carbon number ranging from 18 to 70, for instance polyglyceryl-3 diisostearate, isostearyl lactate, octyl hydroxystearate, octyldodecyl hydroxystearate, diisostearyl malate or glyceryl stearate;
*** triesters comprising at least 35 carbon atoms,** especially comprising between 35 and 70 carbon atoms in total, in particular such as triesters of a tricarboxylic acid, such as triisostearyl citrate, or tridecyl trimellitate, or glycol triesters of monocarboxylic acids such as polyglyceryl-2 triisostearate;
*** tetraesters comprising at least 35 carbon atoms,** especially with a total carbon number ranging from 35 to 70, such as pentaerythritol or polyglycerol tetraesters of a monocarboxylic acid, for instance pentaerythrityl tetrapelargonate, pentaerythrityl tetraisostearate, pentaerythrityl tetraisononanoate, glyceryl tris(2-decyl)tetradecanoate, polyglyceryl-2 tetraisostearate or pentaerythrityl tetrakis(2-decyl)tetradecanoate;
*** polyesters obtained by condensation of an unsaturated fatty acid dimer and/or trimer and of diol,** such as those described in patent application FR 0 853 634, in particular such as dilinoleic acid and 1,4-butanediol. Mention may especially be made in this respect of the polymer sold by Biosynthis under the name Viscoplast 14436H (INCI name: dilinoleic acid/butanediol copolymer), or else copolymers of polyols and of dimer diacids, and esters thereof, such as Hailucent ISDA;
*** esters and polyesters of diol dimer and of monocarboxylic or dicarboxylic acid,** such as esters of diol dimer and of fatty acid and esters of diol dimer and of dicarboxylic acid dimer, in particular which may be obtained from a dicarboxylic acid dimer derived in particular from the dimerization of an unsaturated fatty acid especially of C₈ to C₃₄, especially of C₁₂ to C₂₂, in particular of C₁₆ to C₂₀ and more particularly of C₁₈, such as esters of dilinoleic diacids and of dilinoleic diol dimers, for instance those sold by the company Nippon Fine Chemical under the trade names Lusplan DD-DA5® and DD-DA7®;
*** polyesters resulting from the esterification of at least one triglyceride of hydroxylated carboxylic acid(s) with an aliphatic monocarboxylic acid and with an aliphatic dicarboxylic acid, which is optionally unsaturated,** for instance the succinic acid and isostearic acid castor oil sold under the reference Zenigloss by Zenitech;
* **hydrocarbon-based plant oils** such as fatty acid triglycerides (which are liquid at room temperature), especially of fatty acids containing from 7 to 40 carbon atoms, such as heptanoic or octanoic acid triglycerides or jojoba oil; mention may be made in particular of saturated triglycerides such as caprylic/capric triglycerides and mixtures thereof, for example such as the product sold under the reference Myritol 318 from Cognis, glyceryl triheptanoate, glyceryl trioctanoate, and C₁₈₋₃₆ acid triglycerides such as those sold under the reference Dub TGI 24 by Stéarineries Dubois, and unsaturated triglycerides such as castor oil, olive oil, ximenia oil or pracaxi oil;
*** sucrose esters,** preferably chosen from hydrocarbon esters of sucrose and of a C₂-C₆ carboxylic acid, in particular those chosen from mixtures of esters of acetic acid and of isobutyric acid with sucrose, preferably sucrose diacetate hexakis(2-methylpropanoate), especially the compound whose INCI name is Sucrose acetate isobutyrate (sold especially under the reference Sustane SAIB Food Grade Kosher by the company Eastman Chemicals) and the compounds of INCI name Sucrose polysoyate sold under the reference Crodaderm S by the company Croda, sucrose polybehenate sold under the reference Crodaderm B by the company Croda, sucrose polycottonseedate sold under the reference Crodaderm C by the company Croda; and mixtures thereof.

As regards the oils comprising at least one carbonate function, mention may be made most particularly of dialkyl carbonates, the two alkyl chains possibly being identical or different, such as the caprylyl carbonate sold under the name Cetiol CC® by Cognis, diethylhexyl carbonate, for example sold under the reference Tegosoft DEC by the company Evonik-Goldschmidt Personal Care, and mixtures thereof.

### Apolar non-volatile hydrocarbon-based oils

The composition according to the invention can also comprise at least one apolar non-volatile hydrocarbon-based oil.

These oils may be of plant, mineral or synthetic origin.

For the purposes of the present invention, the term "apolar oil" is intended to mean an oil of which the solubility parameter at 25°C, δₐ, is equal to 0 (J/cm³)^{1/2}.

The definition and calculation of the solubility parameters in the Hansen three-dimensional solubility space are described in the article by C.M. Hansen: The three-dimensional solubility parameters, J. Paint Technol. 39, 105 (1967).

According to this Hansen space:
- δ_{D} characterizes the London dispersion forces derived from the formation of dipoles induced during molecular impacts;
- δₚ characterizes the Debye interaction forces between permanent dipoles and also the Keesom interaction forces between induced dipoles and permanent dipoles;
- δₕ characterizes the specific interaction forces (such as hydrogen bonding, acid/base, donor/acceptor, etc.); and
- δₐ is determined by the equation: δₐ = (δₚ² + δₕ²)½.

The parameters δₚ, δₕ, δ_{D} and δₐ are expressed in (J/cm³)^{½}.

The term "hydrocarbon-based oil" means an oil formed essentially from, or even constituted of, carbon and hydrogen atoms, and optionally oxygen and nitrogen atoms, and not containing any silicon or fluorine atoms. It may contain alcohol, ester, ether, carboxylic acid, amine and/or amide groups.

Preferably, the non-volatile apolar hydrocarbon-based oil may be chosen from linear or branched hydrocarbons of mineral or synthetic origin, such as:
- liquid paraffin or derivatives thereof,
- squalane,
- isoeicosane,
- naphthalene oil,
- polybutenes such as Indopol H-100 (molar mass or MW = 965 g/mol), Indopol H-300 (MW = 1340 g/mol) and Indopol H-1500 (MW = 2160 g/mol) sold or manufactured by the company Amoco,
- polyisobutenes, hydrogenated polyisobutenes such as Parleam® sold by the company Nippon Oil Fats, Panalane H-300 E sold or manufactured by the company Amoco (MW = 1340 g/mol), Viseal 20000 sold or manufactured by the company Synteal (MW = 6000 g/mol) and Rewopal PIB 1000 sold or manufactured by the company Witco (MW = 1000 g/mol), or alternatively Parleam Lite sold by NOF Corporation,
- decene/butene copolymers, polybutene/polyisobutene copolymers, in particular Indopol L-14,
- polydecenes and hydrogenated polydecenes such as: Puresyn 10 (MW = 723 g/mol) and Puresyn 150 (MW = 9200 g/mol) sold or manufactured by the company Mobil Chemicals, or alternatively Puresyn 6 sold by ExxonMobil Chemical,
- and mixtures thereof.

In accordance with a first particular embodiment of the present invention, the non-volatile second oil(s) are chosen from non-volatile phenyl silicone oils.

Preferably, the phenyl silicone oil(s) are chosen from phenyl trimethicones, phenyl dimethicones, phenyltrimethylsiloxydiphenylsiloxanes, diphenyl dimethicones, diphenylmethyldiphenyltrisiloxanes, 2-phenylethyl trimethylsiloxysilicates and trimethylsiloxyphenyl dimethicones, and mixtures thereof.

According to a preferred embodiment, the phenyl silicone oil may be chosen from trimethylsiloxyphenyl dimethicones.

In accordance with a second particular embodiment of the present invention, the non-volatile second oil(s) are chosen from non-volatile hydrocarbon-based oils, other than the first oils (E).

Preferably, the hydrocarbon-based oil(s) are chosen from apolar oils, in particular hydrogenated or non-hydrogenated poly(iso)butenes.

According to a third particular embodiment of the invention, the composition comprises at least one phenyl silicone second oil and at least one hydrocarbon-based oil.

More advantageously, the content of non-volatile second oils (F) represents from 5% to 75% by weight, in particular from 5% to 60% by weight and more particularly from 10% to 50% by weight, relative to the total weight of the composition.

According to a particularly preferred embodiment, a composition according to the invention may comprise a total content of non-volatile oils (i.e. all the non-volatile oils of the composition, irrespective of their nature) of between 20% and 80% by weight and preferably between 40% and 75% by weight, relative to the total weight of the composition.

According to a particularly preferred embodiment, the non-volatile oils (i.e. all the non-volatile oils of the composition, irrespective of their nature) and the alkylcellulose are used in the composition according to the invention in a non-volatile oil(s)/alkylcellulose weight ratio of between 0.3 and 20, preferably between 1 and 15 and better still between 5 and 10.

### VOLATILE ADDITIONAL OILS

The composition according to the invention may also comprise at least one additional volatile oil.

Preferably, the said volatile oil is chosen from volatile hydrocarbon-based oils, volatile silicone oils and/or volatile fluoro oils.

The additional volatile oil may especially be a silicone oil, a hydrocarbon-based oil, which is preferably apolar, or a fluoro oil.

According to a first embodiment, the additional volatile oil is a silicone oil and may be chosen especially from silicone oils with a flash point ranging from 40°C to 102°C, preferably with a flash point of greater than 55°C and less than or equal to 95°C, and preferentially ranging from 65°C to 95°C.

As additional volatile silicone oils that may be used in the invention, mention may be made of linear or cyclic silicones with a viscosity at room temperature of less than 8 centistokes (cSt) (8 × 10⁻⁶ m²/s), and especially containing from 2 to 10 silicon atoms and in particular from 2 to 7 silicon atoms, these silicones optionally comprising alkyl or alkoxy groups containing from 1 to 10 carbon atoms. As volatile silicone oils that may be used in the invention, mention may be made especially of dimethicones with viscosities of 5 and 6 cSt, octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane and dodecamethylpentasiloxane, and mixtures thereof.

According to a second embodiment, the additional volatile oil is a fluoro oil, such as nonafluoromethoxybutane or perfluoromethylcyclopentane, and mixtures thereof.

According to a third embodiment, the additional volatile oil is a hydrocarbon-based oil, which is preferably apolar.

The additional apolar volatile hydrocarbon-based oil may have a flash point ranging from 40°C to 102°C, preferably ranging from 40°C to 55°C and preferentially ranging from 40°C to 50°C.

The additional hydrocarbon-based volatile oil may especially be chosen from hydrocarbon-based volatile oils containing from 8 to 16 carbon atoms, and mixtures thereof, and especially:
- branched C₈-C₁₆ alkanes such as C₈-C₁₆ isoalkanes (also known as isoparaffins), isododecane, isodecane and isohexadecane, and, for example, the oils sold under the trade name Isopar or Permethyl,
- linear alkanes, for instance n-dodecane (C12) and n-tetradecane (C14) sold by Sasol under the respective references Parafol 12-97 and Parafol 14-97, and also mixtures thereof, the undecane-tridecane mixture (Cetiol UT), the mixtures of n-undecane (C11) and of n-tridecane (C13) obtained in Examples 1 and 2 of patent application WO 2008/155 059 from the company Cognis, and mixtures thereof.

According to a particular embodiment, the additional volatile oil(s) may be present in a content ranging from 0.1% to 30% by weight and especially from 0.5% to 20% by weight, relative to the total weight of the said composition.

Advantageously, the composition contains less than 10% by weight of monoalcohols containing from 1 to 5 carbon atoms, and preferably less than 5% by weight.

According to one particular embodiment, the composition may be free of monoalcohols containing from 1 to 5 carbon atoms.

According to a preferred embodiment, the composition is free of additional volatile oil.

### HYDROPHILIC GELLING AGENTS

The composition according to the invention comprises at least one hydrophilic gelling agent chosen from optionally modified polymers of natural origin.

For the purposes of the present patent application, the term "hydrophilic gelling agent" or "polymer for gelling the aqueous phase" means a polymer that is capable of gelling the aqueous phase of the compositions according to the invention.

The gelling polymer that may be used according to the invention may in particular be characterized by its capacity to form in water, beyond a certain concentration C*, a gel characterized by oscillatory rheology (µ = 1 Hz) by a flow threshold τ_{c} at least equal to 10 Pa. This concentration C* may vary widely according to the nature of the gelling polymer under consideration.

In particular, the said polymer is chosen from:
- **galactomannans and derivatives thereof,** such as locust bean gum, fenugreek gum, Cassia gum and guar gums, and derivatives thereof.

As regards the guar gum derivatives, nonionic guar gums modified with C₁-C₆ hydroxyalkyl groups are more particularly intended. Mention may be made, among the hydroxyalkyl groups, by way of example, of the hydroxymethyl, hydroxyethyl, hydroxypropyl and hydroxybutyl groups. These guar gums are well known from the state of the art and can, for example, be prepared by reacting the corresponding alkene oxides, such as, for example, propylene oxides, with the guar gum, so as to obtain a guar gum modified by hydroxypropyl groups. The degree of hydroxyalkylation, which corresponds to the number of alkylene oxide molecules consumed by the number of free hydroxyl functional groups present on the guar gum, preferably varies from 0.4 to 1.2.

Such nonionic guar gums optionally modified with hydroxyalkyl groups are sold, for example, under the trade names Jaguar HP8, Jaguar HP60 and Jaguar HP120, Jaguar DC 293 and Jaguar HP 105 by the company Rhône-Poulenc (Meyhall) or under the name Galactasol 4H4FD2 by the company Aqualon.

Use may also be made of ionic alkyl guar derivatives, and particularly hydroxypropyl guar, modified with sodium methylcarboxylate groups (for example the product sold under the reference Jaguar XC97-1 by Rhodia), hydroxypropyltrimethylammonium guar chloride, and mixtures thereof;
- **biopolysaccharide gums of microbial origin,** such as scleroglucan gum or xanthan gum.

The xanthan gums are especially sold under the names Keltrol, Keltrol T, Keltrol TF, Keltrol BT, Keltrol RD and Keltrol CG by the company Nutrasweet Kelco, or under the names Rhodicare S and Rhodicare H by the company Rhodia Chimie;
- **gums derived from plant exudates,** such as gum arabic, ghatti gum, karaya gum, gum tragacanth, carrageenan gum, agar gum and locust bean gum;
- **pectins, alginates, starches;**
- **mixtures thereof.**

Preferably, the composition comprises at least one hydrophilic gelling agent chosen from guar gums and derivatives thereof, preferably chosen from guar gum, hydroxypropyl guar, hydroxypropyl guar modified with sodium methylcarboxylate groups, hydroxypropyltrimethylammonium guar chloride and xanthan gum, and mixtures thereof; preferably hydroxypropyl guar.

In accordance with a particularly advantageous embodiment of the invention, the content of hydrophilic gelling agent represents from 0.1% to 20% by weight, preferably from 0.1% to 10% by weight and more particularly from 0.1% to 5% by weight, relative to the weight of the composition.

### C₂-C₈ POLYOL

In accordance with another characteristic of the invention, the composition comprises at least one saturated or unsaturated, linear or branched C₂-C₈ and preferably C₃-C₆ polyol, comprising from 2 to 6 hydroxyl groups.

Preferably, the polyol is chosen from glycerol, propylene glycol, 1,3-butylene glycol, dipropylene glycol and diglycerol, and mixtures thereof.

Preferably, the polyol content represents from 0.1% to 20% by weight, preferably from 1% to 10% by weight and more particularly from 2% to 6% by weight, relative to the total weight of the composition.

### SURFACTANTS

The composition according to the invention may contain one or more surfactants that are present, for example, in a content ranging from 0.1% to 20% by weight, or even 0.5% to 15% by weight and preferably from 1% to 10% by weight, relative to the total weight of the composition.

A surfactant appropriately chosen to obtain an oil-in-water emulsion is preferably used.

These surfactants may be chosen from nonionic, anionic, cationic and amphoteric surfactants, and mixtures thereof, preferably nonionic surfactants.

Reference may be made to Kirk-Othmer's Encyclopedia of Chemical Technology, Volume 22, pp. 333-432, 3rd Edition, 1979, Wiley, for the definition of the emulsifying properties and functions of surfactants, in particular pp. 347-377 of this reference, for the anionic, amphoteric and nonionic surfactants.

According to a first embodiment, the composition comprises at least one hydrocarbon-based surfactant.

### Nonionic surfactants

The nonionic surfactants may in particular be chosen from alkyl and polyalkyl esters of poly(ethylene oxide), oxyalkylenated alcohols, alkyl and polyalkyl ethers of poly(ethylene oxide), optionally polyoxyethylenated alkyl and polyalkyl esters of sorbitan, optionally polyoxyethylenated alkyl and polyalkyl ethers of sorbitan, alkyl and polyalkyl glycosides or polyglycosides, in particular alkyl and polyalkyl glucosides or polyglucosides, alkyl and polyalkyl esters of sucrose, optionally polyoxyethylenated alkyl and polyalkyl esters of glycerol, and optionally polyoxyethylenated alkyl and polyalkyl ethers of glycerol, and mixtures thereof.
1) Alkyl and polyalkyl esters of poly(ethylene oxide) that are preferably used are those with a number of ethylene oxide (EO) units ranging from 2 to 200. Examples that may be mentioned include stearate 40 EO, stearate 50 EO, stearate 100 EO, laurate 20 EO, laurate 40 EO and distearate 150 EO.
2) Alkyl and polyalkyl ethers of poly(ethylene oxide) that are preferably used are those with a number of ethylene oxide (EO) units ranging from 2 to 200. Examples that may be mentioned include cetyl ether 23 EO, oleyl ether 50 EO and phytosterol 30 EO.
3) As oxyalkylenated alcohols, which are in particular oxyethylenated and/or oxypropylenated, use is preferably made of fatty alcohols, especially comprising at least 8 carbon atoms, and which may comprise from 1 to 150 oxyethylene and/or oxypropylene units, in particular containing from 20 to 100 oxyethylene units; in particular ethoxylated fatty alcohols, especially of C₈-C₂₄ and preferably of C₁₂-C₁₈, such as stearyl alcohol ethoxylated with 10 oxyethylene units (CTFA name Stearate-10), stearyl alcohol ethoxylated with 20 oxyethylene units (CTFA name Steareth-20), for instance Brij 78 sold by the company Uniqema, stearyl alcohol ethoxylated with 100 oxyethylene units (CTFA name Steareth-100), cetearyl alcohol ethoxylated with 30 oxyethylene units (CTFA name Ceteareth-30), and the mixture of C₁₂-C₁₅ fatty alcohols comprising 7 oxyethylene units (CTFA name C₁₂-C₁₅ Pareth-7), for instance the product sold under the name Neodol 25-7® by Shell Chemicals; behenyl alcohol ethoxylated with 100 oxyethylene units (CTFA name Beheneth-100) or in particular oxyalkylenated (oxyethylenated and/or oxypropylenated) alcohols containing from 1 to 15 oxyethylene and/or oxypropylene units, in particular ethoxylated C₈-C₂₄ and preferably C₁₂-C₁₈ fatty alcohols, such as stearyl alcohol ethoxylated with 2 oxyethylene units (CTFA name Steareth-2), for instance Brij 72 sold by the company Uniqema; stearyl alcohol ethoxylated with 20 oxyethylene units (CTFA name Steareth-20), for instance Brij 78 sold by the company Uniqema.
4) Optionally polyoxyethylenated alkyl and polyalkyl esters of sorbitan that are preferably used include those with a number of ethylene oxide (EO) units ranging from 0 to 100. Examples that may be mentioned include sorbitan laurate 4 or 20 EO, in particular polysorbate 20 (or polyoxyethylene (20) sorbitan monolaurate) such as the product Tween 20 sold by the company Uniqema, sorbitan palmitate 20 EO, sorbitan stearate 20 EO, sorbitan oleate 20 EO, or the Cremophor products (RH 40, RH 60, etc.) from BASF. Non-polyoxyethylenated sorbitan alkyl and polyalkyl esters that may preferably be used include a mixture of sorbitan stearate and of sucrose cocoate, preferably the product sold under the reference Arlacel 2121u from Croda.
5) Optionally polyoxyethylenated alkyl and polyalkyl ethers of sorbitan that are preferably used include those with a number of ethylene oxide (EO) units ranging from 0 to 100.
6) Alkyl and polyalkyl glucosides or polyglucosides that are preferably used are those containing an alkyl group comprising from 6 to 30 carbon atoms and preferably from 6 to 18 or even from 8 to 16 carbon atoms, and containing a glucoside group preferably comprising from 1 to 5 and in particular 1, 2 or 3 glucoside units. The alkylpolyglucosides may be chosen, for example, from decylglucoside (alkyl-C₉/C₁₁-polyglucoside (1.4)), for instance the product sold under the name Mydol 10® by the company Kao Chemicals or the product sold under the name Plantacare 2000 UP® by the company Henkel and the product sold under the name Oramix NS 10® by the company SEPPIC; caprylyl/capryl glucoside, for instance the product sold under the name Plantacare KE 3711® by the company Cognis or Oramix CG 110® by the company SEPPIC; laurylglucoside, for instance the product sold under the name Plantacare 1200 UP® by the company Henkel or Plantaren 1200 N® by the company Henkel; cocoglucoside, for instance the product sold under the name Plantacare 818 UP® by the company Henkel; caprylylglucoside, for instance the product sold under the name Plantacare 810 UP® by the company Cognis; and mixtures thereof.
   More generally, the surfactants of alkylpolyglycoside type are defined more specifically hereinbelow.
7) Examples of alkyl and polyalkyl esters of sucrose that may be mentioned are Crodesta F150, sucrose monolaurate sold under the name Crodesta SL 40, and the products sold by Ryoto Sugar Ester, for instance sucrose palmitate sold under the reference Ryoto Sugar Ester P1670, Ryoto Sugar Ester LWA1695 or Ryoto Sugar Ester 01570.
8) Optionally polyoxyethylenated alkyl and polyalkyl esters of glycerol that are preferably used are those with a number of ethylene oxide (EO) units ranging from 0 to 100 and a number of glycerol units ranging from 1 to 30. Examples that may be mentioned include hexaglyceryl monolaurate, PEG-30 glyceryl stearate and glyceryl stearate.
9) Optionally polyoxyethylenated alkyl and polyalkyl ethers of glycerol that are preferably used include those with a number of ethylene oxide (EO) units ranging from 0 to 100 and a number of glycerol units ranging from 1 to 30. Examples that may be mentioned include Nikkol Batyl Alcohol 100 and Nikkol Chimyl Alcohol 100.

### Anionic surfactants

The anionic surfactants may be chosen from alkyl (ether) sulfates, carboxylates, amino acid derivatives, sulfonates, isethionates, taurates, sulfosuccinates, alkylsulfoacetates, phosphates and alkyl phosphates, polypeptides, metal salts of C₁₀-C₃₀ and in particular C₁₂-C₂₀ fatty acids, in particular metal stearates, and mixtures thereof.
1) As alkyl sulfates and alkyl ether sulfates of alkali metals, for example containing at least 12 carbon atoms and more particularly from 12 to 16 carbon atoms, and optionally comprising from 1 to 20 oxyethylene units, more particularly from 1 to 10 oxyethylene units, examples that may be mentioned include sodium lauryl sulfate, sodium lauryl ether sulfate (70/30 C12-14) (2.2 EO) sold under the names Sipon AOS225 or Texapon N702 by the company Henkel, ammonium lauryl ether sulfate (70/30 C₁₂₋₁₄) (3 EO) sold under the name Sipon LEA 370 by the company Henkel, ammonium (C₁₂-C₁₄) alkyl ether (9 EO) sulfate sold under the name Rhodapex AB/20 by the company Rhodia Chimie, and the mixture of sodium magnesium lauryl oleyl ether sulfate sold under the name Empicol BSD 52 by the company Albright & Wilson.
2) Examples of carboxylates that may be mentioned include salts (for example alkali metal salts) of N-acylamino acids, glycol carboxylates, amido ether carboxylates (AEC) and polyoxyethylenated carboxylic acid salts.

The surfactant of glycol carboxylate type may be chosen from alkyl glycol carboxylics or 2-(2-hydroxyalkyloxy acetate), salts thereof and mixtures thereof. These alkyl glycol carboxylics comprise a linear or branched, saturated or unsaturated aliphatic and/or aromatic alkyl chain containing from 8 to 18 carbon atoms. These carboxylics may be neutralized with mineral bases such as potassium hydroxide or sodium hydroxide.

Examples of surfactants of glycol carboxylic type that may be mentioned include sodium lauryl glycol carboxylate or sodium 2-(2-hydroxyalkyloxy acetate) such as the product sold under the name Beaulight Shaa® by the company Sanyo, Beaulight LCA-25N® or the corresponding acid form Beaulight Shaa (Acid form)®.

An example of an amido ether carboxylate (AEC) that may be mentioned is sodium lauryl amido ether carboxylate (3 EO) sold under the name Akypo Foam 30® by the company Kao Chemicals.

Examples of polyoxyethylenated carboxylic acid salts that may be mentioned include oxyethylenated (6 EO) sodium lauryl ether carboxylate (65/25/10 C₁₂₋₁₄₋₁₆) sold under the name Akypo Soft 45 NV® by the company Kao Chemicals, polyoxyethylenated and carboxymethylated fatty acids of olive oil origin sold under the name Olivem 400® by the company Biologia e Tecnologia, and oxyethylenated (6 EO) sodium tridecyl ether carboxylate sold under the name Nikkol ECTD-6 NEX® by the company Nikkol.
3) Amino acid derivatives that may especially be mentioned include alkali metal salts of amino acids, such as:
   - sarcosinates, for instance the sodium lauroyl sarcosinate sold under the name Sarkosyl NL 97® by the company Ciba or sold under the name Oramix L30® by the company SEPPIC, sodium myristoyl sarcosinate sold under the name Nikkol Sarcosinate MN® by the company Nikkol, and sodium palmitoyl sarcosinate sold under the name Nikkol Sarcosinate PN® by the company Nikkol;
   - alaninates, for instance sodium N-lauroyl N-methyl amidopropionate sold under the name Sodium Nikkol Alaninate LN30® by the company Nikkol, or sold under the name Alanone ALE® by the company Kawaken, and triethanolamine N-lauroyl N-methyl alanine sold under the name Alanone Alta® by the company Kawaken;
   - glutamates, for instance triethanolamine monococoyl glutamate sold under the name Acylglutamate CT-12® by the company Ajinomoto, or triethanolamine lauroyl glutamate sold under the name Acylglutamate LT-12® by the company Ajinomoto;
   - aspartates, for instance the mixture of triethanolamine N-lauroyl aspartate and of triethanolamine N-myristoyl aspartate, sold under the name Asparack® by the company Mitsubishi;
   - glycine derivatives (glycinates), for instance the sodium N-cocoyl glycinate sold under the names Amilite GCS-12® and Amilite GCK 12 by the company Ajinomoto;
   - citrates, such as the oxyethylenated (9 mol) citric monoester of cocoyl alcohols sold under the name Witconol EC 1129 by the company Goldschmidt, and the citric acid monoester of glyceryl stearate;
   - galacturonates, such as the sodium dodecyl-D-galactoside uronate sold by the company Soliance.
4) Examples of sulfonates that may be mentioned include α-olefin sulfonates, for instance the sodium α-olefin sulfonate (C₁₄₋₁₆) sold under the name Bio-Terge AS 40® by the company Stepan, sold under the names Witconate AOS Protégé® and Sulframine AOS PH 12® by the company Witco or sold under the name Bio-Terge AS 40 CG® by the company Stepan, the sodium secondary olefin sulfonate sold under the name Hostapur SAS 30® by the company Clariant.
5) Isethionates that may be mentioned include acylisethionates, for instance sodium cocoylisethionate, such as the product sold under the name Jordapon CI P® by the company Jordan.
6) Taurates that may be mentioned include the sodium salt of palm kernel oil methyltaurate sold under the name Hostapon CT Pate® by the company Clariant; N-acyl N-methyltaurates, for instance the sodium N-cocoyl N-methyltaurate sold under the name Hostapon LT-SF® by the company Clariant or sold under the name Nikkol CMT-30-T® by the company Nikkol, and the sodium palmitoyl methyltaurate sold under the name Nikkol PMT® by the company Nikkol.
7) Examples of sulfosuccinates that may be mentioned include the oxyethylenated (3 EO) lauryl alcohol monosulfosuccinate (70/30 C₁₂/C₁₄) sold under the names Setacin 103 Special® and Rewopol SB-FA 30 K 4® by the company Witco, the disodium salt of a C₁₂-C₁₄ alkyl hemisulfosuccinate, sold under the name Setacin F Special Paste® by the company Zschimmer Schwarz, the oxyethylenated (2 EO) disodium oleamidosulfosuccinate sold under the name Standapol SH 135® by the company Henkel, the oxyethylenated (5 EO) laurylamide monosulfosuccinate sold under the name Lebon A-5000® by the company Sanyo, the oxyethylenated (10 EO) disodium salt of lauryl citrate monosulfosuccinate sold under the name Rewopol SB CS 50® by the company Witco, and the ricinoleic monoethanolamide monosulfosuccinate sold under the name Rewoderm S 1333® by the company Witco. Polydimethylsiloxane sulfosuccinates may also be used, such as disodium PEG-12 dimethicone sulfosuccinate sold under the name Mackanate-DC30 by the company MacIntyre.
8) Examples of alkyl sulfoacetates that may be mentioned include the mixture of sodium lauryl sulfoacetate and disodium lauryl ether sulfosuccinate, sold under the name Stepan-Mild LSB by the company Stepan.
9) Examples of phosphates and alkyl phosphates that may be mentioned include monoalkyl phosphates and dialkyl phosphates, such as the lauryl monophosphate sold under the name MAP 20® by the company Kao Chemicals, the potassium salt of dodecylphosphoric acid, mixture of monoester and diester (predominantly diester) sold under the name Crafol AP-31® by the company Cognis, the mixture of octylphosphoric acid monoester and diester sold under the name Crafol AP-20® by the company Cognis, the mixture of ethoxylated (7 mol of EO) phosphoric acid monoester and diester of 2-butyloctanol, sold under the name Isofol 12 7 EO-Phosphate Ester® by the company Condea, the potassium or triethanolamine salt of mono(C₁₂-C₁₃)alkyl phosphate sold under the references Arlatone MAP 230K-40® and Arlatone MAP 230T-60® by the company Uniqema, the potassium lauryl phosphate sold under the name Dermalcare MAP XC-99/09® by the company Rhodia Chimie, and the potassium cetyl phosphate sold under the name Arlatone MAP 160K by the company Uniqema.
10) The polypeptides are obtained, for example, by condensation of a fatty chain onto amino acids from cereals and in particular from wheat and oat. Examples of polypeptides that may be mentioned include the potassium salt of hydrolysed lauroyl wheat protein, sold under the name Aminofoam W OR by the company Croda, the triethanolamine salt of hydrolysed cocoyl soybean protein, sold under the name May-Tein SY by the company Maybrook, the sodium salt of lauroyl oat amino acids, sold under the name Proteol Oat by the company SEPPIC, collagen hydrolysate grafted onto coconut fatty acid, sold under the name Geliderm 3000 by the company Deutsche Gelatine, and soybean proteins acylated with hydrogenated coconut acids, sold under the name Proteol VS 22 by the company SEPPIC.
11) As metal salts of C₁₀-C₃₀ and especially C₁₂-C₂₀ fatty acids, mention may be made in particular of metal stearates, such as sodium stearate and potassium stearate, and also polyhydroxystearates.

### Cationic surfactants

The cationic surfactants may be chosen from:
- alkylimidazolidiniums such as isostearylethylimidonium ethosulfate,
- ammonium salts such as (C₁₂₋₃₀ alkyl)tri(C₁₋₄ alkyl)ammonium halides, for instance N,N,N-trimethyl-1-docosanaminium chloride (or behentrimonium chloride).

### Amphoteric surfactants

The compositions according to the invention may also contain one or more amphoteric surfactants, for instance N-acylamino acids such as N-alkyl aminoacetates and disodium cocoamphodiacetate, and amine oxides such as stearamine oxide, or alternatively silicone surfactants, for instance dimethicone copolyol phosphates such as the product sold under the name Pecosil PS 100® by the company Phoenix Chemical.

Preferably, the composition according to the invention comprises at least one nonionic surfactant as stabilizer, preferably chosen from alkyl and polyalkyl esters of sorbitan, which are in particular non-polyoxyethylenated, and better still a mixture of sorbitan stearate and of sucrose cocoate, preferably the product sold under the reference Arlacel 2121 from Croda; or alternatively chosen from fatty alcohols, especially containing at least 8 carbon atoms, which are oxyalkylenated, in particular oxyethylenated and/or oxypropylenated and which may comprise from 1 to 150 oxyethylene and/or oxypropylene units, in particular containing from 20 to 100 oxyethylene units, in particular ethoxylated fatty alcohols, especially of C₈-C₂₄ and preferably of C₁₂-C₁₈, or mixtures thereof.

### Gemini surfactant

According to a particularly advantageous variant of the invention, the composition comprises at least one gemini surfactant of formula (I): 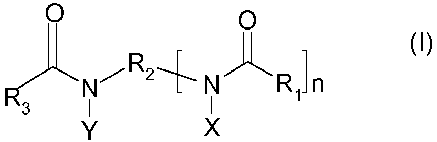 in which:
- R₁ and R₃ denote, independently of each other, an alkyl radical containing from 1 to 25 carbon atoms;
- R₂ denotes a spacer group consisting of a linear or branched alkylene chain containing from 1 to 12 carbon atoms;
- X and Y denote, independently of each other, a group -(C₂H₄O)ₐ-(C₃H₆O)_{b}Z, in which
   - Z denotes a hydrogen atom or a radical -CH₂-COOM, -SO₃M, -P(O)(OM)₂, -C₂H₄-SO₃M, -C₃H₆-SO₃M or -CH₂(CHOH)₄CH₂OH, where M and M' represent H or an alkali metal or alkaline-earth metal or ammonium or alkanolammonium ion,
   - a ranges from 0 to 15, b ranges from 0 to 10, and the sum of a + b ranges from 1 to 25; and
   - n ranges from 1 to 10.

The gemini surfactant of formula (I) is preferably such that each of the groups R₁-CO- and R₃-CO- comprises from 8 to 20 carbon atoms, and preferably denotes a coconut fatty acid residue (mainly comprising lauric acid and myristic acid).

In addition, this surfactant is preferably such that, for each of the X and Y radicals, the sum of a and b has an average value ranging from 10 to 20 and is preferably equal to 15. A preferred group for Z is the group -SO₃M, where M is preferably an alkali metal ion, such as a sodium ion.

The spacer group R₂ advantageously consists of a linear C₁-C₃ alkylene chain, and preferably an ethylene (CH₂CH₂) chain.

Finally, n is advantageously equal to 1.

A surfactant of this type is in particular the one identified by the INCI name: Sodium dicocoylethylenediamine PEG-15 sulfate, having the following structure: 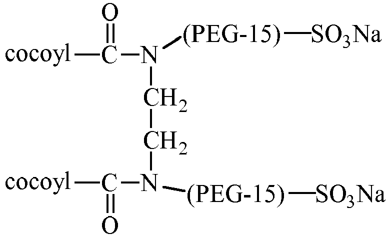 it being understood that PEG represents the group CH₂CH₂O and cocoyl represents the coconut fatty acid residue.

This surfactant has a molecular structure very similar to that of ceramide-3.

Preferably, the gemini surfactant according to the invention is used as a mixture with other surfactants, and especially as a mixture with (a) an ester of a C₆-C₂₂ fatty acid (preferably C₁₄-C₂₀ such as a stearate) and of glyceryl, (b) a diester of a C₆-C₂₂ fatty acid (preferably C₁₄-C₂₀ such as a stearate) and of citric acid and of glycerol (especially a diester of a C₆-C₂₂ fatty acid and of glyceryl monocitrate), and (c) a C₁₀-C₃₀ fatty alcohol (preferably behenyl alcohol).

Advantageously, the composition according to the invention comprises a mixture of
sodium dicocoylethylenediamine PEG-15 sulfate, of glyceryl stearate, of glyceryl stearate monocitrate, of behenyl alcohol.

More preferentially, the gemini surfactant according to the invention represents from 10% to 20% by weight and advantageously 15% by weight; the glyceryl ester of a C₆-C₂₂ fatty acid represents from 30% to 40% by weight, advantageously 35% by weight; the diester of a C₆-C₂₂ fatty acid and of citric acid and of glycerol represents from 10% to 20% by weight, advantageously 15% by weight; and the C₁₀-C₃₀ fatty alcohol represents from 30% to 40% by weight, advantageously 35% by weight, relative to the total weight of the mixture of surfactants containing the gemini surfactant.

Advantageously, the composition according to the invention comprises a mixture of from 10% to 20% by weight of sodium dicocoylethylenediamine PEG-15 sulfate, from 30% to 40% by weight of glyceryl stearate, from 10% to 20% by weight of glyceryl stearate monocitrate and from 30% to 40% by weight of behenyl alcohol, relative to the total weight of the mixture of surfactants comprising the gemini surfactant.

As a variant, the gemini surfactant according to the invention may be used as a mixture with an anionic surfactant, such as an ester of lauric acid, sodium lauroyl lactate. In this case, the gemini surfactant preferably represents from 30% to 50% by weight and the anionic surfactant represents from 50% to 70% by weight, relative to the total weight of the mixture.

The gemini surfactant may be used, for example, as a mixture with other surfactants in the form of the products sold by Sasol under the name Ceralution®, and especially the following products:
- Ceralution® H: Behenyl Alcohol, Glyceryl Stearate, Glyceryl Stearate Citrate and Sodium Dicocoylethylenediamine PEG-15 Sulfate,
- Ceralution® F: Sodium Lauroyl Lactylate and Sodium Dicocoylethylenediamine PEG-15 Sulfate,
- Ceralution® C: Aqua, Capric/Caprylic triglyceride, Glycerin, Ceteareth-25, Sodium Dicocoylethylenediamine PEG-15 Sulfate, Sodium Lauroyl Lactylate, Behenyl Alcohol, Glyceryl Stearate, Glyceryl Stearate Citrate, Gum Arabic, Xanthan Gum, Phenoxyethanol, Methylparaben, Ethylparaben, Butylparaben, Isobutylparaben (INCI names).

This gemini surfactant represents from 3% to 50% of the weight of these mixtures.

Preferably, the composition comprises as gemini surfactant the compound whose INCI name is behenyl alcohol, glyceryl stearate, glyceryl stearate citrate and sodium dicocoylethylenediamine PEG-15 sulfate, sold under the name Ceralution® H by the company Sasol.

In accordance with a preferred embodiment of the invention, the composition comprises at least one anionic surfactant and at least one nonionic surfactant as defined previously.

According to a more particular variant of this embodiment, the composition also comprises at least one gemini surfactant of formula (I) detailed above.

More particularly, the content of anionic surfactant(s) present in the composition according to the invention represents from 0.1% to 2% by weight and preferably from 0.2% to 1.5% by weight, relative to the weight of the composition.

Preferably, the content of nonionic surfactant(s) present in the composition according to the invention represents from 0.5% to 10% by weight and preferably from 1% to 5% by weight, relative to the weight of the composition.

Preferably, if the composition comprises any, the content of gemini surfactant of formula (I) is between 0.1% and 3% by weight and even more particularly between 0.2% and 2% by weight, relative to the total weight of the composition.

### ADDITIONAL COMPOUNDS

A composition according to the invention may also comprise at least one additional compound chosen from fillers, solid fatty substances, semi-crystalline polymers and film-forming polymers, and mixtures thereof.

Preferably, the additional compound is featured by at least one solid fatty substance.

Preferably, the solid fatty substances are chosen from waxes and pasty fatty substances, and mixtures thereof.

### Fillers

A cosmetic composition used according to the invention may comprise at least one filler, of organic or mineral nature.

The term "filler" should be understood as meaning colourless or white, solid particles of any form, which are in a form that is insoluble and dispersed in the medium of the composition. These particles, of mineral or organic nature, can give body or rigidity to the composition and/or softness and uniformity to the makeup. They are different from colorants.

Among the fillers that may be used in the compositions according to the invention, mention may be made of silica, kaolin, bentone, starch, lauroyllysine, and fumed silica particles, optionally hydrophilically or hydrophobically treated, and mixtures thereof.

A composition used according to the invention may comprise one or more fillers in a content ranging from 0.1% to 15% by weight and in particular from 1% to 10% by weight relative to the total weight of the composition.

### Wax(es)

Advantageously, a solid fatty substance is featured by at least one wax. Thus, the composition according to the invention may also comprise at least one wax.

For the purposes of the invention, the term "wax" means a lipophilic compound, which is solid at room temperature (25°C), with a reversible solid/liquid change of state, which has a melting point of greater than or equal to 30°C, which may be up to 120°C.

The waxes that may be used in a composition according to the invention are chosen from solid waxes that may or may not be deformable at room temperature, of animal, plant, mineral or synthetic origin, and mixtures thereof.

Examples that may be mentioned include solid fatty alcohols comprising from 10 to 24 carbon atoms and more preferentially from 12 to 22 carbon atoms. As particular examples of fatty alcohols that may preferably be used, mention may be made especially of myristyl alcohol, palmityl alcohol, behenyl alcohol, erucyl alcohol and arachidyl alcohol, and mixtures thereof.

Hydrocarbon-based waxes, for instance beeswax, lanolin wax or Chinese insect wax; rice wax, carnauba wax, candelilla wax, ouricury wax, esparto grass wax, cork fibre wax, sugar cane wax, Japan wax and sumach wax; montan wax, microcrystalline waxes, paraffins and ozokerite; polyethylene waxes, the waxes obtained by Fisher-Tropsch synthesis and waxy copolymers, and also esters thereof, may especially be used.

Mention may also be made of waxes obtained by catalytic hydrogenation of animal or plant oils containing linear or branched C₈-C₃₂ fatty chains.

Among these waxes that may especially be mentioned are hydrogenated jojoba oil, hydrogenated sunflower oil, hydrogenated castor oil, hydrogenated coconut oil, hydrogenated lanolin oil, bis(1,1,1-trimethylolpropane) tetrastearate sold under the name Hest 2T-4S by the company Heterene, and bis(1,1,1-trimethylolpropane) tetrabehenate sold under the name Hest 2T-4B by the company Heterene.

The waxes obtained by transesterification and hydrogenation of plant oils, such as castor oil or olive oil, for instance the waxes sold under the names Phytowax ricin 16L64® and 22L73® and Phytowax Olive 18L57 by the company Sophim, may also be used. Such waxes are described in patent application FR-A-2 792 190.

It is also possible to use silicone waxes, which may advantageously be substituted polysiloxanes, preferably of low melting point.

Among the commercial silicone waxes of this type, mention may be made especially of those sold under the names Abilwax 9800, 9801 or 9810 (Goldschmidt), KF910 and KF7002 (Shin-Etsu), or 176-1118-3 and 176-11481 (General Electric).

The silicone waxes that may be used may also be alkyl or alkoxy dimethicones such as the following commercial products: Abilwax 2428, 2434 and 2440 (Goldschmidt), or VP 1622 and VP 1621 (Wacker), and also (C₂₀-C₆₀) alkyl dimethicones, in particular (C₃₀-C₄₅) alkyl dimethicones, such as the silicone wax sold under the name SF-1642 by the company GE-Bayer Silicones.

It is also possible to use hydrocarbon-based waxes modified with silicone or fluoro groups, for instance: siliconyl candelilla, siliconyl beeswax and fluoro beeswax from Koster Keunen.

The waxes may also be chosen from fluoro waxes.

According to a preferred embodiment, a composition according to the invention may comprise at least one wax with a melting point of less than 65°C and preferably less than 63°C.

Such a wax may be chosen in particular from paraffin wax, stearyl alcohol, hydrogenated cocoglycerides, synthetic beeswax (especially the product sold under the reference Cyclochem 326 A by Evonik-Goldschmidt), palm butter, sumach wax, silicone-treated beeswax, stearyl stearate, alkyl dimethicone wax, certain polymethylene waxes (such as Cirebelle 303 sold by Cirebelle), berry wax, olive oil wax, lemon wax, ceresin wax (sold under the reference White Ceresin Wax JH 520 by the company Hansotech), and mixtures thereof.

According to an even more preferred embodiment, a composition according to the invention may comprise at least one hydrogenated olive oil ester wax, preferably the product whose INCI name is Hydrogenated stearyl olive esters, for instance the product sold under the reference Phytowax Olive 18 L 57 from Sophim and the product whose INCI name is Hydrogenated myristoyl olive esters, especially such as the product sold under the reference Phytowax Olive 14L48 from Sophim.

According to one embodiment, the composition according to the invention is free of wax.

According to another embodiment, the composition according to the invention may comprise a content of additional wax(es) with a melting point of less than 65°C ranging from 0.1% to 10% by weight and better still from 0.5% to 5% by weight, relative to the total weight of the composition.

In particular, the composition according to the invention may comprise a total content of wax of between 0.1% and 30% by weight, relative to the total weight of the composition.

Preferably, the composition according to the invention may comprise between 0.1% and 20% by weight of wax(es), more particularly between 0.5% and 15% by weight and better still between 1% and 10% by weight, relative to the total weight of the composition.

Preferably, when the composition is in liquid form, it comprises a total content of waxes of between 0.1% and 5% by weight, relative to the total weight of the composition.

### Pasty fatty substances

Advantageously, the solid fatty substance is featured by a pasty fatty substance. This embodiment is particularly advantageous when it is desired to obtain a glossy or satiny deposit, for instance in the case of a lip makeup product, for instance a lipstick.

Thus, the composition under consideration according to the invention may also comprise at least one pasty fatty substance.

For the purposes of the present invention, the term "pasty fatty substance" means a lipophilic fatty compound that undergoes a reversible solid/liquid change in state, which exhibits, in the solid state, an anisotropic crystalline arrangement and which comprises, at a temperature of 23°C, a liquid fraction and a solid fraction.

In other words, the starting melting point of the pasty compound can be less than 23°C. The liquid fraction of the pasty compound, measured at 23°C, can represent from 9% to 97% by weight of the compound. This liquid fraction at 23°C preferably represents between 15% and 85% and more preferably between 40% and 85% by weight.

For the purposes of the invention, the melting point corresponds to the temperature of the most endothermic peak observed in thermal analysis (DSC) as described in the standard ISO 11357-3; 1999. The melting point of a paste or of a wax may be measured using a differential scanning calorimeter (DSC), for example the calorimeter sold under the name MDSC 2920 by the company TA Instruments.

The measurement protocol is as follows:
A sample of 5 mg of paste or wax (depending on the case) placed in a crucible is subjected to a first temperature rise passing from -20°C to 100°C, at the heating rate of 10°C/minute, then is cooled from 100°C to -20°C at a cooling rate of 10°C/minute and finally subjected to a second temperature rise passing from -20°C to 100°C at a heating rate of 5°C/minute. During the second temperature rise, the variation in the difference between the power absorbed by the empty crucible and the crucible containing the sample of paste or wax as a function of the temperature is measured. The melting point of the compound is the value of the temperature corresponding to the top of the peak of the curve representing the variation in the difference in power absorbed as a function of the temperature.

The liquid fraction by weight of the pasty compound at 23°C is equal to the ratio of the heat of fusion consumed at 23°C to the heat of fusion of the pasty compound.

The heat of fusion of the pasty compound is the heat consumed by the compound in order to pass from the solid state to the liquid state. The pasty compound is said to be in the solid state when all of its mass is in crystalline solid form. The pasty compound is said to be in the liquid state when all of its mass is in liquid form.

The heat of fusion of the pasty compound is equal to the area under the curve of the thermogram obtained using a differential scanning calorimeter (DSC), such as the calorimeter sold under the name MDSC 2920 by TA Instruments, with a temperature rise of 5°C or 10°C per minute, according to Standard ISO 11357-3:1999. The heat of fusion of the pasty compound is the amount of energy required to make the compound change from the solid state to the liquid state. It is expressed in J/g.

The heat of fusion consumed at 23°C is the amount of energy absorbed by the sample to change from the solid state to the state that it has at 23°C, composed of a liquid fraction and a solid fraction.

The liquid fraction of the pasty compound measured at 32°C preferably represents from 30% to 100% by weight of the compound, preferably from 50% to 100% by weight and more preferably from 60% to 100% by weight of the compound. When the liquid fraction of the pasty compound measured at 32°C is equal to 100%, the temperature of the end of the melting range of the pasty compound is less than or equal to 32°C.

The liquid fraction of the pasty compound measured at 32°C is equal to the ratio of the heat of fusion consumed at 32°C to the heat of fusion of the pasty compound. The heat of fusion consumed at 32°C is calculated in the same way as the heat of fusion consumed at 23°C.

The pasty fatty substance may be chosen from synthetic compounds and compounds of plant origin. A pasty fatty substance may be obtained by synthesis from starting materials of plant origin.

The pasty fatty substance is advantageously chosen from:
- lanolin and derivatives thereof, such as lanolin alcohol, oxyethylenated lanolins, acetylated lanolin, lanolin esters such as isopropyl lanolate, and oxypropylenated lanolins,
- polymeric or non-polymeric silicone compounds, for instance polydimethylsiloxanes of high molecular masses, polydimethylsiloxanes containing side chains of the alkyl or alkoxy type containing from 8 to 24 carbon atoms, especially stearyl dimethicones,
- polymeric or non-polymeric fluoro compounds,
- vinyl polymers, especially:
   - olefin homopolymers,
   - olefin copolymers,
   - hydrogenated diene homopolymers and copolymers,
   - linear or branched oligomers, homopolymers or copolymers of alkyl (meth)acrylates preferably containing a C₈-C₃₀ alkyl group,
   - oligomers, homopolymers and copolymers of vinyl esters containing C₈-C₃₀ alkyl groups,
   - oligomers, homopolymers and copolymers of vinyl ethers containing C₈-C₃₀ alkyl groups,
   - liposoluble polyethers resulting from the polyetherification between one or more C₂-C₁₀₀ and preferably C₂-C₅₀ diols,
- esters and polyesters, and
- mixtures thereof,

The pasty fatty substance may be a polymer, especially a hydrocarbon-based polymer.

A preferred silicone and fluoro pasty fatty substance is polymethyltrifluoropropylmethylalkyldimethylsiloxane, manufactured under the name X22-1088 by Shin-Etsu.

Among the liposoluble polyethers, mention may be made especially of copolymers of ethylene oxide and/or of propylene oxide with C₆-C₃₀ alkylene oxides. Preferably, the weight ratio of the ethylene oxide and/or propylene oxide to the alkylene oxides in the copolymer is from 5/95 to 70/30. In this family, mention will be made especially of block copolymers comprising C₆-C₃₀ alkylene oxide blocks with a molecular weight ranging from 1000 to 10 000, for example a polyoxyethylene/polydodecylene glycol block copolymer such as the ethers of dodecanediol (22 mol) and of polyethylene glycol (45 oxyethylene or OE units) sold under the brand name Elfacos ST9 by Akzo Nobel.

Among the esters, the following are especially preferred:
- esters of a glycerol oligomer, especially diglycerol esters, in particular condensates of adipic acid and of glycerol, for which some of the hydroxyl groups of the glycerols have reacted with a mixture of fatty acids such as stearic acid, capric acid, stearic acid and isostearic acid, and 12-hydroxystearic acid, for instance those sold under the brand name Softisan 649 by the company Sasol; or alternatively which have reacted with a mixture of fatty acids such as lauric acid, palmitic acid, cetylic acid and stearic acid, for instance those sold under the reference Softisan 100 by the company Cremer Oleo;
- phytosterol esters;
- pentaerythritol esters;
- esters formed from:
   - at least one C₁₆₋₄₀ alcohol, at least one of the alcohols being a Guerbet alcohol, and
   - a diacid dimer formed from at least one unsaturated C₁₈₋₄₀ fatty acid,
such as the ester of a dimer of fatty acids and of tall oil comprising 36 carbon atoms and of a mixture i) of Guerbet alcohols comprising 32 carbon atoms and ii) of behenyl alcohol; the ester of a dimer of linoleic acid and of a mixture of two Guerbet alcohols, 2-tetradecyloctadecanol (32 carbon atoms) and 2-hexadecyleicosanol (36 carbon atoms);
- non-crosslinked polyesters resulting from the polycondensation between a linear or branched C₄-C₅₀ dicarboxylic acid or polycarboxylic acid and a C₂-C₅₀ diol or polyol;
- polyesters resulting from the esterification between a polycarboxylic acid and an ester of aliphatic hydroxylated carboxylic acid, such as Risocast DA-Lglycer and Risocast DA-H sold by the Japanese company Kokyu Alcohol Kogyo, which are esters resulting from the esterification reaction of hydrogenated castor oil with dilinoleic acid or isostearic acid; and
- aliphatic esters of an ester resulting from the esterification between an ester of aliphatic hydroxylated carboxylic acid and an aliphatic carboxylic acid, for example the product sold under the trade name Salacos HCIS (V)-L by the company Nisshin Oil.

A Guerbet alcohol is the reaction product of the Guerbet reaction, which is well known to those skilled in the art. It is a reaction for transforming a primary aliphatic alcohol into its β-alkyl dimeric alcohol with loss of one equivalent of water.

The aliphatic carboxylic acids described above generally comprise from 4 to 30 and preferably from 8 to 30 carbon atoms. They are preferably chosen from hexanoic acid, heptanoic acid, octanoic acid, 2-ethylhexanoic acid, nonanoic acid, decanoic acid, undecanoic acid, dodecanoic acid, tridecanoic acid, tetradecanoic acid, pentadecanoic acid, hexadecanoic acid, hexyldecanoic acid, heptadecanoic acid, octadecanoic acid, isostearic acid, nonadecanoic acid, eicosanoic acid, isoarachidic acid, octyldodecanoic acid, heneicosanoic acid and docosanoic acid, and mixtures thereof.

The aliphatic carboxylic acids are preferably branched.

The esters of aliphatic hydroxylated carboxylic acid are advantageously derived from an aliphatic hydroxylated carboxylic acid comprising from 2 to 40 carbon atoms, preferably from 10 to 34 carbon atoms and better still from 12 to 28 carbon atoms, and from 1 to 20 hydroxyl groups, preferably from 1 to 10 hydroxyl groups and better still from 1 to 6 hydroxyl groups. The esters of aliphatic hydroxylated carboxylic acid are especially chosen from:
a) partial or total esters of saturated linear monohydroxylated aliphatic monocarboxylic acids;
b) partial or total esters of unsaturated monohydroxylated aliphatic monocarboxylic acids;
c) partial or total esters of saturated monohydroxylated aliphatic polycarboxylic acids;
d) partial or total esters of saturated polyhydroxylated aliphatic polycarboxylic acids;
e) partial or total esters of C₂ to C₁₆ aliphatic polyols that have reacted with a monohydroxylated or polyhydroxylated aliphatic monocarboxylic or polycarboxylic acid,
f) and mixtures thereof;
   - esters of a diol dimer and of a diacid dimer, where appropriate esterified on their free alcohol or acid function(s) with acid or alcohol radicals, especially dimer dilinoleate esters; such esters may be chosen especially from the esters having the following INCI nomenclature: bis-behenyl/isostearyl/phytosteryl dimer dilinoleyl dimer dilinoleate (Plandool G), phytosteryl/isostearyl/stearyl/behenyl dimer dilinoleate (Plandool H or Plandool S), and mixtures thereof;
   - butters of plant origin, such as mango butter, such as the product sold under the name Lipex 203 by the company Aarhuskarlshamn, shea butter, in particular the product whose INCI name is Butyrospermum Parkii Butter, such as the product sold under the reference Sheasoft® by the company Aarhuskarlshamn, cupuacu butter (Rain Forest RF3410 from the company Beraca Sabara), murumuru butter (Rain Forest RF3710 from the company Beraca Sabara), cocoa butter; and also orange wax, for instance the product sold under the reference Orange Peel Wax by the company Koster Keunen;
   - totally or partially hydrogenated plant oils, for instance hydrogenated soybean oil, hydrogenated coconut oil, hydrogenated rapeseed oil, mixtures of hydrogenated plant oils such as the mixture of hydrogenated soybean, coconut, palm and rapeseed plant oil, for example the mixture sold under the reference Akogel® by the company Aarhuskarlshamn (INCI name Hydrogenated Vegetable Oil), the trans-isomerized partially hydrogenated jojoba oil manufactured or sold by the company Desert Whale under the commercial reference Iso-Jojoba-50®, partially hydrogenated olive oil, for instance the compound sold under the reference Beurrolive by the company Soliance.

The aliphatic esters of an ester are advantageously chosen from:
- the ester resulting from the esterification reaction of hydrogenated castor oil with isostearic acid in proportions of 1 to 1 (1/1), known as hydrogenated castor oil monoisostearate,
- the ester resulting from the esterification reaction of hydrogenated castor oil with isostearic acid in proportions of 1 to 2 (1/2), known as hydrogenated castor oil diisostearate,
- the ester resulting from the esterification reaction of hydrogenated castor oil with isostearic acid in proportions of 1 to 3 (1/3), known as hydrogenated castor oil triisostearate,
- and mixtures thereof.

In particular, the composition according to the invention may comprise a total content of pasty fatty substance of between 0.1% and 30% by weight relative to the total weight of the composition.

Preferably, the pasty fatty substance(s) may be present in an amount ranging from 0.5% to 30% by weight and especially from 1% to 20% by weight, relative to the total weight of the composition.

### ADDITIONAL MOISTURIZERS

According to a first embodiment, the composition may also comprise at least one additional moisturizer (also known as a humectant) other than glycerol.

Additional moisturizers or humectants that may especially be mentioned include sorbitol, urea and derivatives thereof, especially Hydrovance (2-hydroxyethyl urea) sold by National Starch, lactic acids, hyaluronic acid, AHAs, BHAs, sodium pidolate, xylitol, serine, sodium lactate, ectoin and derivatives thereof, chitosan and derivatives thereof, collagen, plankton, an extract of Imperata cylindra sold under the name Moist 24 by the company Sederma, acrylic acid homopolymers, for instance Lipidure-HM® from NOF Corporation, β-glucan and in particular sodium carboxymethyl β-glucan from Mibelle-AG-Biochemistry; a mixture of passionflower oil, apricot oil, corn oil and rice bran oil sold by Nestlé under the name NutraLipids® ; a C-glycoside derivative such as those described in patent application WO 02/051 828 and in particular C-β-D-xylopyranoside-2-hydroxypropane in the form of a solution containing 30% by weight of active material in a water/propylene glycol mixture (60/40% by weight) such as the product manufactured by Chimex under the trade name Mexoryl SBB®; an oil of musk rose sold by Nestlé; an extract of the microalga Prophyridium cruentum enriched with zinc, sold by Vincience under the name Algualane Zinc®, spheres of collagen and of chondroitin sulfate of marine origin (Atelocollagen) sold by the company Engelhard Lyon under the name Marine Filling Spheres; hyaluronic acid spheres such as those sold by the company Engelhard Lyon; and arginine.

Use will preferably be made of a moisturizer chosen from glycerol, urea and derivatives thereof, especially Hydrovance® sold by National Starch, hyaluronic acid, AHAs, BHAs, acrylic acid homopolymers, for instance Lipidure-HM® from NOF Corporation, β-glucan and in particular sodium carboxymethyl β-glucan from Mibelle-AG-Biochemistry; a mixture of passionflower oil, apricot oil, corn oil and rice bran oil sold by Nestlé under the name NutraLipids®; a C-glycoside derivative such as those described in patent application WO 02/051 828 and in particular C-β-D-xylopyranoside-2-hydroxypropane in the form of a solution containing 30% by weight of active material in a water/propylene glycol mixture (60/40% by weight) such as the product manufactured by Chimex under the trade name Mexoryl SBB®; an oil of musk rose sold by Nestlé; an extract of the microalga Prophyridium cruentum enriched with zinc, sold by Vincience under the name Algualane Zinc®; spheres of collagen and of chondroitin sulfate of marine origin (Atelocollagen) sold by the company Engelhard Lyon under the name Marine Filling Spheres; hyaluronic acid spheres such as those sold by the company Engelhard Lyon; and arginine.

### AQUEOUS PHASE

As stated above, a composition according to the invention comprises at least 5% by weight of water and preferably at least 10% by weight of water, relative to the total weight of the composition.

Preferably, a composition according to the invention may comprise from 5% to 80% by weight, preferably from 10% to 70% by weight and in particular from 15% to 50% by weight of water, relative to its total weight.

The composition in accordance with the invention may comprise, besides water, at least one water-soluble solvent.

The aqueous phase may constitute the continuous phase of the composition.

The term "composition with an aqueous continuous phase" means that the composition has a conductivity, measured at 25°C, of greater than or equal to 23 µS/cm (microSiemens/cm), the conductivity being measured, for example, using an MPC227 conductimeter from Mettler Toledo and an Inlab730 conductivity measuring cell. The measuring cell is immersed in the composition so as to remove the air bubbles that might be formed between the two electrodes of the cell. The conductivity reading is taken once the conductimeter value has stabilized. A mean is determined on at least three successive measurements.

In the present invention, the term "water-soluble solvent" denotes a compound that is liquid at room temperature and water-miscible (miscibility with water of greater than 50% by weight at 25°C and atmospheric pressure).

The water-soluble solvents that may be used in the compositions according to the invention may also be volatile.

Among the water-soluble solvents that may be used in the compositions in accordance with the invention, mention may be made especially of lower monoalcohols containing from 1 to 5 carbon atoms, such as ethanol and isopropanol, C₃ and C₄ ketones and C₂-C₄ aldehydes.

Preferably, the composition according to the invention comprises a total content of monoalcohols comprising between 2 and 8 carbon atoms of between 0 and 10% by weight (limits inclusive) relative to the total weight of the composition.

Preferably, the composition according to the invention comprises a total content of monoalcohols comprising between 2 and 8 carbon atoms of between 0 and 5% by weight (limits inclusive) relative to the total weight of the composition.

Preferably, the composition according to the invention is free of monoalcohols comprising between 2 and 8 carbon atoms.

Preferably, the said monoalcohol(s) comprising between 2 and 8 carbon atoms are chosen from ethanol, butanol, methanol and isopropanol.

However, the content of aqueous phase indicated previously does not include the contents of each of the abovementioned compounds.

A composition according to the invention may also comprise any additional component usually used in cosmetics, such as dyestuffs, fillers or cosmetic active agents.

Needless to say, a person skilled in the art will take care to select the optional additional compounds and/or the amount thereof such that the advantageous properties of the composition used according to the invention are not, or are not substantially, adversely affected by the envisaged addition.

### DYESTUFFS

A composition according to the invention preferably comprises at least one dyestuff. Preferably, it is chosen from water-soluble or water-insoluble, liposoluble or non-liposoluble, organic or mineral dyestuffs, and materials with an optical effect, and mixtures thereof.

For the purposes of the present invention, the term "dyestuff means a compound that is capable of producing a coloured optical effect when it is formulated in sufficient amount in a suitable cosmetic medium.

According to a preferred embodiment, a composition according to the invention comprises at least one water-soluble dyestuff.

The water-soluble dyestuffs used according to the invention are more particularly water-soluble dyes.

For the purposes of the invention, the term "water-soluble dye" means any natural or synthetic, generally organic compound, which is soluble in an aqueous phase or water-miscible solvents and which is capable of colouring. In particular, the term "water-soluble" means the capacity of a compound to be dissolved in water, measured at 25°C, to a concentration at least equal to 0.1 g/l (production of a macroscopically isotropic, transparent, coloured or colourless solution). This solubility is in particular greater than or equal to 1 g/l.

As water-soluble dyes that are suitable for use in the invention, mention may be made especially of synthetic or natural water-soluble dyes, for instance FDC Red 4 (CI: 14700), DC Red 6 (Lithol Rubine Na; CI: 15850), DC Red 22 (CI: 45380), DC Red 28 (CI: 45410 Na salt), DC Red 30 (CI: 73360), DC Red 33 (CI: 17200), DC Orange 4 (CI: 15510), FDC Yellow 5 (CI: 19140), FDC Yellow 6 (CI: 15985), DC Yellow 8 (CI: 45350 Na salt), FDC Green 3 (CI: 42053), DC Green 5 (CI: 61570), FDC Blue 1 (CI: 42090).

As non-limiting illustrations of sources of water-soluble dyestuff(s) that may be used in the context of the present invention, mention may be made in particular of those of natural origin, such as extracts of cochineal carmine, of beetroot, of grape, of carrot, of tomato, of annatto, of paprika, of henna, of caramel and of curcumin.

Thus, the water-soluble dyestuffs that are suitable for use in the invention are in particular carminic acid, betanin, anthocyans, enocyanins, lycopene, β-carotene, bixin, norbixin, capsanthin, capsorubin, flavoxanthin, lutein, cryptoxanthin, rubixanthin, violaxanthin, riboflavin, rhodoxanthin, cantaxanthin and chlorophyll, and mixtures thereof.

They may also be copper sulfate, iron sulfate, water-soluble sulfopolyesters, rhodamine, betaine, methylene blue, the disodium salt of tartrazine and the disodium salt of fuchsin.

Some of these water-soluble dyestuffs are in particular permitted for food use. Representatives of these dyes that may be mentioned more particularly include dyes of the carotenoid family, referenced under the food codes E120, E162, E163, E160a-g, E150a, E101, E100, E140 and E141.

According to a preferred variant, the water-soluble dyestuff(s) that are to be transferred onto the skin and/or the lips intended to be made up are formulated in a physiologically acceptable medium so as to be compatible with impregnation into the substrate.

The water-soluble dyestuff(s) may be present in a composition according to the invention in a content ranging from 0.01% to 8% by weight and preferably from 0.1% to 6% by weight, relative to the total weight of the said composition.

According to a particularly preferred embodiment, the water-soluble dyestuffs(s) are chosen from the disodium salt of brilliant yellow FCF sold by the company LCW under the name DC Yellow 6, the disodium salt of fuchsin acid D sold by the company LCW under the name DC Red 33, and the trisodium salt of Rouge Allura sold by the company LCW under the name FD & C Red 40.

According to a particular embodiment of the invention, the composition according to the invention comprises only water-soluble dyes as dyestuffs.

According to another embodiment, a composition according to the invention may comprise, besides the water-soluble dyestuffs described previously, one or more additional dyestuffs, especially such as pigments or nacres, conventionally used in cosmetic compositions.

The term "pigments" should be understood as meaning white or coloured, inorganic (mineral) or organic particles, which are insoluble in the liquid organic phase, and which are intended to colour and/or opacify the composition and/or the deposit produced with the composition.

The pigments may be chosen from mineral pigments, organic pigments and composite pigments (i.e. pigments based on mineral and/or organic materials).

The pigments may be chosen from monochromatic pigments, lakes, nacres, and pigments with an optical effect, for instance reflective pigments and goniochromatic pigments.

The mineral pigments may be chosen from metal oxide pigments, chromium oxides, iron oxides, titanium dioxide, zinc oxides, cerium oxides, zirconium oxides, manganese violet, Prussian blue, ultramarine blue and ferric blue, and mixtures thereof.

The organic pigments may be, for example:
- cochineal carmine,
- organic pigments of azo dyes, anthraquinone dyes, indigoid dyes, xanthene dyes, pyrene dyes, quinoline dyes, triphenylmethane dyes or fluoran dyes,
- organic lakes or insoluble sodium, potassium, calcium, barium, aluminium, zirconium, strontium or titanium salts of acidic dyes such as azo, anthraquinone, indigoid, xanthene, pyrene, quinoline, triphenylmethane or fluoran dyes. These dyes generally comprise at least one carboxylic or sulfonic acid group,
- melanin-based pigments.

Among the organic pigments, mention may be made of D&C Blue No. 4, D&C Brown No. 1, D&C Green No. 5, D&C Green No. 6, D&C Orange No. 4, D&C Orange No. 5, D&C Orange No. 10, D&C Orange No. 11, D&C Red No. 6, D&C Red No. 7, D&C Red No. 17, D&C Red No. 21, D&C Red No. 22, D&C Red No. 27, D&C Red No. 28, D&C Red No. 30, D&C Red No. 31, D&C Red No. 33, D&C Red No. 34, D&C Red No. 36, D&C Violet No. 2, D&C Yellow No. 7, D&C Yellow No. 8, D&C Yellow No. 10, D&C Yellow No. 11, FD&C Blue No. 1, FD&C Green No. 3, FD&C Red No. 40, FD&C Yellow No. 5 and FD&C Yellow No. 6.

The hydrophobic treatment agent may be chosen from silicones such as methicones, dimethicones and perfluoroalkylsilanes; fatty acids such as stearic acid; metal soaps such as aluminium dimyristate, the aluminium salt of hydrogenated tallow glutamate, perfluoroalkyl phosphates, perfluoroalkylsilanes, perfluoroalkylsilazanes, polyhexafluoropropylene oxides, polyorganosiloxanes comprising perfluoroalkyl perfluoropolyether groups, amino acids; N-acylamino acids or salts thereof; lecithin, isopropyl triisostearyl titanate, and mixtures thereof.

The N-acylamino acids can comprise an acyl group having from 8 to 22 carbon atoms, such as, for example, a 2-ethylhexanoyl, caproyl, lauroyl, myristoyl, palmitoyl, stearoyl or cocoyl group. The salts of these compounds may be aluminium, magnesium, calcium, zirconium, zinc, sodium or potassium salts. The amino acid can, for example, be lysine, glutamic acid or alanine.

The term "alkyl" mentioned in the compounds cited above denotes in particular an alkyl group having from 1 to 30 carbon atoms and preferably having from 5 to 16 carbon atoms.

Hydrophobic-treated pigments are described especially in patent application EP-A-1 086 683.

For the purposes of the present patent application, the term "nacre" means coloured particles of any form, which may or may not be iridescent, especially produced by certain molluscs in their shell, or alternatively synthesized, and which have a colour effect via optical interference.

Examples of nacres that may be mentioned include nacreous pigments such as titanium mica coated with an iron oxide, mica coated with bismuth oxychloride, titanium mica coated with chromium oxide, titanium mica coated with an organic dye in particular of the abovementioned type, and also nacreous pigments based on bismuth oxychloride. They may also be mica particles, at the surface of which are superposed at least two successive layers of metal oxides and/or of organic dyestuffs.

The nacres may more particularly have a yellow, pink, red, bronze, orangey, brown, gold and/or coppery colour or tint.

As illustrations of nacres that may be introduced as interference pigments into the first composition, mention may be made of the gold-coloured nacres sold in particular by the company Engelhard under the name Brilliant gold 212G (Timica), Gold 222C (Cloisonne), Sparkle gold (Timica), Gold 4504 (Chromalite) and Monarch gold 233X (Cloisonne); the bronze nacres sold in particular by the company Merck under the name Bronze fine (17384) (Colorona) and Bronze (17353) (Colorona) and by the company Engelhard under the name Super bronze (Cloisonne); the orange nacres sold in particular by the company Engelhard under the name Orange 363C (Cloisonne) and Orange MCR 101 (Cosmica) and by the company Merck under the name Passion orange (Colorona) and Matte orange (17449) (Microna); the brown nacres sold in particular by the company Engelhard under the name Nu-antique copper 340XB (Cloisonne) and Brown CL4509 (Chromalite); the nacres with a copper tint sold in particular by the company Engelhard under the name Copper 340A (Timica); the nacres with a red tint sold in particular by the company Merck under the name Sienna fine (17386) (Colorona); the nacres with a yellow tint sold in particular by the company Engelhard under the name Yellow (4502) (Chromalite); the red nacres with a gold tint sold in particular by the company Engelhard under the name Sunstone G012 (Gemtone); the pink nacres sold in particular by the company Engelhard under the name Tan opale G005 (Gemtone); the black nacres with a gold tint sold in particular by the company Engelhard under the name Nu antique bronze 240 AB (Timica), the blue nacres sold in particular by the company Merck under the name Matte blue (17433) (Microna), the white nacres with a silvery tint sold in particular by the company Merck under the name Xirona Silver, and the golden-green pink-orange nacres sold in particular by the company Merck under the name Indian summer (Xirona), and mixtures thereof.

### USUAL ADDITIONAL COSMETIC INGREDIENTS

A composition according to the invention may also comprise any common cosmetic ingredient, which may be chosen especially from antioxidants, additional film-forming polymers (lipophilic or hydrophilic) other than alkylcellulose and in particular other than ethylcellulose, fragrances, preserving agents, neutralizers, sunscreens, sweeteners, vitamins, free-radical scavengers and sequestrants, and mixtures thereof.

Needless to say, a person skilled in the art will take care to select the optional additional ingredients and/or the amount thereof such that the advantageous properties of the composition according to the invention are not, or are not substantially, adversely affected by the envisaged addition.

According to a particular embodiment, a composition according to the invention may advantageously comprise:
- from 4% to 30% by weight of alkylcellulose, preferably ethylcellulose,
- from 15% to 60% by weight of water,
- from 0.1% to 20% by weight of hydrophilic gelling agent(s), advantageously chosen from galactomannans and preferably guar or a derivative thereof,
- from 0.1% to 20% by weight of glycerol,
- preferably from 0.1% to 3% by weight of gemini surfactant of formula (I),
- from 5% to 75% by weight of a non-volatile hydrocarbon-based first oil, and even more preferentially a C₁₀-C₂₆ alcohol (E),
- from 5% to 60% by weight of non-volatile silicone oils (F).

A composition according to the invention may more particularly be a composition for making up and/or caring for keratin materials, in particular the skin and/or the lips, and better still the lips.

A composition according to the invention may constitute a liquid lipstick for the lips, a body makeup product, a facial or body care product or an antisun product.

According to one preferred embodiment, a composition of the invention is in liquid form. As illustrations of liquid formulations, mention may be made especially of lip glosses and/or more generally liquid lipsticks.

According to a preferred embodiment, a composition of the invention is a liquid lipstick.

The composition according to the invention may be manufactured via the known processes generally used in cosmetics or dermatology.

Throughout the description, including the claims, the term "comprising a" should be understood as being synonymous with "comprising at least one", unless otherwise specified.

The terms "between... and..." and "ranging from... to..." should be understood as being inclusive of the limits, unless otherwise specified.

The examples and figures that follow are presented as nonlimiting illustrations of the invention.

### EXAMPLE 1

The following liquid lip composition was prepared, the ingredients of which have been collated in the table below (the contents are expressed as weight percentages of active material, unless otherwise indicated):

| **Compounds (Chemical Name/Commercial Reference)** | **Content** |
|---|---|
| Ethylcellulose (dispersion in water - 30% solids, containing ethylcellulose, sodium lauryl sulfate, cetyl alcohol; Aquacoat ECD 30 from FMC Biopolymer) | 24* |
| Octyldodecanol | 24.6 |
| Trimethylsiloxyphenyl Dimethicone (Belsil PDM 1000 from Wacker) | 21.5 |
| Behenyl alcohol (and) glyceryl stearate (and) disodium ethylene dicocamide PEG-15 disulfate (and) glyceryl stearate citrate (Ceralution H from Sasol) | 3* |
| Steareth-20 (Brij S20-PA-(SG) from the company Croda) | 1 |
| Glycerol | 3 |
| Polybutene (Indopol H 1500 from Ineos) | 3 |
| Polymethylene wax (Cirebelle 505 from Cirebelle) | 6 |
| Hydroxypropyl guar (Jaguar HP 105 from Rhodia) | 0.2 |
| Cetyl alcohol | 4 |
| Preserving agent | qs |
| Pigments/nacres | 2.7 |
| Ethanol | 3 |
| Water | qs 100 |

| | |
|---|---|
| * expressed as commercial product | |

### Preparation protocol

1) The aqueous dispersion of ethylcellulose was mixed with water and the hydrocarbon-based non-volatile oil(s) with stirring using a Rayneri blender, and the mixture was heated for 1 to 2 hours at 55°C.
2) The surfactant and then the hydroxypropyl guar and the glycerol were added, and the mixture was stirred at 55°C until homogeneous.
3) The dyes preground in part of the fatty phase were then added.
4) The non-volatile silicone oil was then added, with continued stirring at 55°C, until the mixture was homogeneous, and the heating was then stopped.
5) Finally, the alcohol and the phenoxyethanol were added with stirring.
6) The stirring was then stopped and the mixture was then packaged in heating sachets.
7) The compositions were left to stand for 24 hours at room temperature.

The texture of this composition is particularly creamy and stable.

It was furthermore found that the deposit obtained using this composition was comfortable, and afforded a substantial increase in coverage when compared with a composition not comprising glycerol.

### EXAMPLE 2

The following liquid lip composition was prepared, the ingredients of which have been collated in the table below (the contents are expressed as weight percentages of active material, unless otherwise indicated):

| **Compounds (Chemical Name/Commercial Reference)** | **Content** |
|---|---|
| Ethylcellulose (dispersion in water - 30% solids, containing ethylcellulose, sodium lauryl sulfate, cetyl alcohol; Aquacoat ECD 30 from FMC Biopolymer) | 30.5* |
| Octyldodecanol | 31 |
| Trimethylsiloxyphenyl Dimethicone (Belsil PDM 1000 from Wacker) | 21.3 |
| Sorbitan stearate and sucrose cocoate (Arlacel 2121 U from Croda) | 3.8* |
| Glycerol | 3 |
| Hydroxypropyl guar (Jaguar HP 105 from Rhodia) | 0.2 |
| Ethanol | 3 |
| Pigments | 0.16 |
| Preserving agent | qs |
| Fragrance | qs |
| Water | qs 100 |

| | |
|---|---|
| * expressed as commercial product | |

### Composition preparation protocol:

1) The dyes were ground in part of the fatty phase.
2) The aqueous dispersion of ethylcellulose was mixed with water and octyldodecanol with stirring using a Rayneri blender, and the mixture was heated for 1 to 2 hours at 55°C.
3) The surfactant and then the hydroxypropyl guar and the glycerol were added, and the mixture was stirred at 55°C until homogeneous.
4) The preground dyes were then added.
5) The non-volatile silicone oil was then added, with continued stirring at 55°C, until the mixture was homogeneous, and the heating was then stopped.
6) Finally, the alcohol, the preserving agents and the fragrances were added with stirring.
7) The stirring was then stopped and the mixture was then packaged in heating sachets.
8) The compositions were left to stand for 24 hours at room temperature.

The texture of this composition is particularly creamy and stable.

It was furthermore found that the deposit obtained using this composition was comfortable, and afforded a substantial increase in coverage when compared with a composition not comprising glycerol.

## Claims

1. Cosmetic composition in the form of an oil-in-water emulsion comprising:
(A) at least 5% by weight of water relative to the total weight of the composition;
(B) at least 4% by weight of alkylcellulose, the alkyl residue of which comprises between 2 and 6 carbon atoms;
(C) at least one hydrophilic gelling agent chosen from optionally modified polymers of natural origin;
(D) at least one saturated or unsaturated, linear or branched C₂-C₈ and preferably C₃-C₆ polyol, comprising from 2 to 6 hydroxyl groups;
(E) at least one hydrocarbon-based non-volatile first oil chosen from:
- C₁₀-C₂₆ alcohols, preferably monoalcohols;
- optionally hydroxylated monoesters, diesters or triesters of a C₂-C₈ monocarboxylic or polycarboxylic acid and of a C₂-C₈ alcohol, which are optionally hydroxylated;
- esters of a C₂-C₈ polyol and of one or more C₂-C₈ carboxylic acids; and
(F) at least one non-volatile second oil chosen from silicone oils and/or fluoro oils or hydrocarbon-based oils other than the said first oil.

2. Composition according to the preceding claim, **characterized in that** the alkylcellulose is present in a content ranging from 4% to 60% by weight, preferably from 4% to 50% by weight, better still from 5% to 30% by weight and more preferentially from 5% to 20% by weight, relative to the total weight of the composition.

3. Composition according to either one of the preceding claims, **characterized in that** the alkylcellulose is chosen from ethylcellulose and propylcellulose, and is preferably ethylcellulose.

4. Composition according to any one of the preceding claims, **characterized in that** the hydrocarbon-based non-volatile first oil(s) (E) are chosen from:
- lauryl alcohol, stearyl alcohol, isostearyl alcohol, oleyl alcohol, erucyl alcohol, 2-hexyldecyl alcohol, isocetyl alcohol and octyldodecanol, and mixtures thereof;
- optionally hydroxylated monoesters of a C₂-C₈ carboxylic acid and of a C₂-C₈ alcohol;
- optionally hydroxylated diesters of a C₂-C₈ dicarboxylic acid and of a C₂-C₈ alcohol, such as diisopropyl adipate, 2-diethylhexyl adipate, dibutyl adipate or diisostearyl adipate;
- optionally hydroxylated triesters of a C₂-C₈ tricarboxylic acid and of a C₂-C₈ alcohol, such as citric acid esters, such as trioctyl citrate, triethyl citrate, acetyl tributyl citrate, tributyl citrate or acetyl tributyl citrate;
- esters of a C₂-C₈ polyol and of one or more C₂-C₈ carboxylic acids, such as glycol diesters of monoacids, such as neopentyl glycol diheptanoate, or glycol triesters of monoacids, such as triacetin.

5. Composition according to the preceding claim, **characterized in that** the hydrocarbon-based non-volatile oil(s) (E) are chosen from C₁₀-C₂₆ alcohols, preferably monoalcohols, preferably chosen from lauryl alcohol, isostearyl alcohol, oleyl alcohol, erucyl alcohol, 2-hexyldecyl alcohol, isocetyl alcohol and octyldodecanol, and mixtures thereof, preferably octyldodecanol.

6. Composition according to any one of the preceding claims, **characterized in that** the non-volatile hydrocarbon-based first oil(s) (E) are present in a content ranging from 5% to 75% by weight, in particular from 10% to 50% by weight and preferably from 20% to 45% by weight, relative to the total weight of the composition.

7. Composition according to any one of the preceding claims, **characterized in that** the non-volatile second oil(s) (F) are chosen from phenyl silicone oils, preferably chosen from phenyl trimethicones, phenyl dimethicones, phenyltrimethylsiloxydiphenylsiloxanes, diphenyl dimethicones, diphenyl methyldiphenyl trisiloxanes, 2-phenylethyl trimethylsiloxysilicates, trimethylsiloxyphenyl dimethicones, and mixtures thereof, and preferably trimethylsiloxyphenyl dimethicones.

8. Composition according to any one of the preceding claims, **characterized in that** the hydrocarbon-based non-volatile second oil(s) (F), other than the oils (E), are chosen from:
• ester oils comprising at least 18 carbon atoms, especially such as:
* mono esters comprising at least 18 carbon atoms
* monoesters of a fatty acid comprising at least 18 carbon atoms and of diol
* diesters comprising at least 18 carbon atoms
* hydroxylated monoesters and diesters comprising at least 18 carbon atoms
* triesters comprising at least 35 carbon atoms
* tetraesters comprising at least 35 carbon atoms
* polyesters obtained by condensation of dimer and/or trimer of unsaturated fatty acid and of diol
* esters and polyesters of diol dimer and of monocarboxylic or dicarboxylic acid
* polyesters resulting from the esterification of at least one triglyceride of hydroxylated carboxylic acid(s) with an aliphatic monocarboxylic acid and with an aliphatic dicarboxylic acid, which is optionally unsaturated
* hydrocarbon-based plant oils
* sucrose esters
• oils comprising at least one carbonate function
• mixtures thereof.

9. Composition according to any one of the preceding claims, **characterized in that** it comprises a content ranging from 5% to 75% by weight of non-volatile second oil(s) (F), in particular from 5% to 60% by weight and more particularly from 10% to 50% by weight, relative to the total weight of the composition.

10. Composition according to any one of the preceding claims, **characterized in that** it comprises from 5% to 80% by weight, preferably from 10% to 70% by weight, in particular from 15% to 60% by weight and better still from 20% to 50% by weight of water, relative to its total weight.

11. Composition according to any one of the preceding claims, **characterized in that** the hydrophilic gelling agent is chosen from:
- galactomannans and derivatives thereof, such as locust bean gum, fenugreek gum, Cassia gum and guar gums, and derivatives thereof;
- biopolysaccharide gums of microbial origin, such as scleroglucan gum or xanthan gum;
- gums derived from plant exudates, such as gum arabic, ghatti gum, karaya gum, gum tragacanth, carrageenan gum, agar gum and locust bean gum;
- pectins, alginates, starches;
- mixtures thereof.

12. Composition according to any one of the preceding claims, **characterized in that** the hydrophilic gelling agent is chosen from guar gums and derivatives thereof, preferably chosen from guar gum, hydroxypropyl guar, hydroxypropyl guar modified with sodium methylcarboxylate groups, hydroxypropyltrimethylammonium guar chloride, xanthan derivatives, and mixtures thereof; preferably hydroxypropyl guar.

13. Composition according to any one of the preceding claims, **characterized in that** the content of hydrophilic gelling agent represents from 0.1% to 20% by weight, preferably from 0.1% to 10% by weight and more particularly from 0.1% to 5% by weight, relative to the weight of the composition.

14. Composition according to any one of the preceding claims, **characterized in that** the content of polyol represents from 0.1% to 20% by weight, preferably from 1% to 10% by weight and more particularly from 2% to 6% by weight, relative to the weight of the composition.

15. Composition according to any one of the preceding claims, **characterized in that** it comprises at least one anionic surfactant, preferably chosen from alkali metal alkyl sulfates and alkyl ether sulfates, containing at least 12 carbon atoms, more particularly from 12 to 16 carbon atoms, and optionally comprising from 1 to 20 oxyethylene units, and also mixtures thereof.

16. Composition according to the preceding claim, **characterized in that** the content of anionic surfactant represents from 0.1% to 2% by weight and preferably from 0.2% to 1.5% by weight, relative to the weight of the composition.

17. Composition according to any one of the preceding claims, **characterized in that** it comprises at least one nonionic surfactant, preferably chosen from alkyl and polyalkyl esters of sorbitan, which are preferably non-polyoxyethylenated; fatty alcohols, containing at least 8 carbon atoms, and possibly comprising from 1 to 150 oxyethylene and/or oxypropylene units; mixtures thereof.

18. Composition according to the preceding claim, **characterized in that** the content of nonionic surfactant(s) represents from 0.5% to 10% by weight and preferably from 1% to 5% by weight, relative to the weight of the composition.

19. Composition according to any one of the preceding claims, **characterized in that** it comprises at least one gemini surfactant of formula (I): 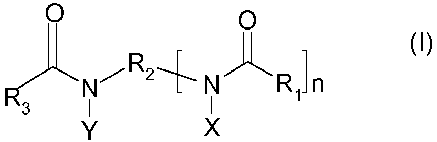 in which:
* R₁ and R₃ denote, independently of each other, an alkyl radical containing from 1 to 25 carbon atoms;
* R₂ denotes a spacer group consisting of a linear or branched alkylene chain containing from 1 to 12 carbon atoms;
* X and Y denote, independently of each other, a group
-(C₂H₄O)ₐ-(C₃H₆O)_{b}Z
in which
* Z denotes a hydrogen atom or a radical -CH₂-COOM, -SO₃M, -P(O)(OM)₂, -C₂H₄-SO₃M, -C₃H₆-SO₃M or -CH₂(CHOH)₄CH₂OH, where M and M' represent H or an alkali metal or alkaline-earth metal or ammonium or alkanolammonium ion,
* a ranges from 0 to 15, b ranges from 0 to 10, and the sum of a + b ranges from 1 to 25; and
* n ranges from 1 to 10.

20. Composition according to the preceding claim, **characterized in that** it comprises a mixture of from 10% to 20% by weight of sodium dicocoylethylenediamine PEG-15 sulfate, from 30% to 40% by weight of glyceryl stearate, from 10% to 20% by weight of glyceryl stearate monocitrate and from 30% to 40% by weight of behenyl alcohol, relative to the total weight of the mixture of surfactants comprising the gemini surfactant.

21. Composition according to either of Claims 19 and 20, **characterized in that** the gemini surfactant of formula (I) is present in a content ranging from 1% to 3% by weight, relative to the total weight of the composition, and preferably ranging from 0.2% to 2% by weight relative to the weight of the composition.

22. Composition according to any one of the preceding claims, **characterized in that** it comprises at least one dyestuff.

23. Composition according to any one of the preceding claims, **characterized in that** the said composition is a composition for making up and/or caring for keratin materials, in particular the lips.

24. Cosmetic process for making up and/or caring for keratin materials, in particular the lips, comprising at least one step that consists in applying to the said keratin materials at least one composition as defined according to any one of the preceding claims.

## Patentansprüche

1. Kosmetische Zusammensetzung in der Form einer Ölin-Wasser-Emulsion, umfassend:
(A) wenigstens 5 Gew.-% Wasser bezogen auf das Gesamtgewicht der Zusammensetzung;
(B) wenigstens 4 Gew.-% Alkylcellulose, deren Alkylrest zwischen 2 und 6 Kohlenstoffatome umfasst;
(C) wenigstens ein hydrophobes Geliermittel ausgewählt aus gegebenenfalls modifizierten Polymeren mit natürlichem Ursprung;
(D) wenigstens ein gesättigtes oder ungesättigtes, lineares oder verzweigtes C₂-C₈- und vorzugsweise C₃-C₆Polyol, das 2 bis 6 Hydroxygruppen umfasst;
(E) wenigstens ein nichtflüchtiges erstes Öl auf Kohlenwasserstoffbasis ausgewählt aus:
- C₁₀-C₂₆Alkoholen, vorzugsweise Monoalkoholen;
- gegebenenfalls hydroxylierten Monoestern, Diestern oder Triestern einer C₂-C₈Monocarbonsäure oder Polycarbonsäure und eines C₂-C₈Alkohols, die gegebenenfalls hydroxyliert sind;
- Estern eines C₂-C₈Polyols und einer oder mehrerer C₂-C₈Carbonsäuren; und
(F) wenigstens ein nichtflüchtiges zweites Öl ausgewählt aus Siliconölen und/oder Fluorölen oder von dem ersten Öl verschiedenen Ölen auf Kohlenwasserstoffbasis.

2. Zusammensetzung gemäß dem vorstehenden Anspruch, **dadurch gekennzeichnet, dass** die Alkylcellulose mit einem Gehalt in dem Bereich von 4 Gew.-% bis 60 Gew.-%, vorzugsweise von 4 Gew.-% bis 50 Gew.-%, noch besser von 5 Gew.-% bis 30 Gew.-% und bevorzugter von 5 Gew.-% bis 20 Gew.-%, bezogen auf das Gesamtgewicht der Zusammensetzung vorhanden ist.

3. Zusammensetzung gemäß einem der vorstehenden Ansprüche, **dadurch gekennzeichnet, dass** die Alkylcellulose ausgewählt ist aus Ethylcellulose und Propylcellulose und vorzugsweise Ethylcellulose ist.

4. Zusammensetzung gemäß einem der vorstehenden Ansprüche, **dadurch gekennzeichnet, dass** die nichtflüchtigen ersten Öl(e) auf Kohlenwasserstoffbasis (E) ausgewählt sind aus:
- Laurylalkohol, Stearylalkohol, Isostearylalkohol, Oleylalkohol, Erucylalkohol, 2-Hexyldecylalkohol, Isocetylalkohol und Octyldodecanol und Gemischen davon;
- gegebenenfalls hydroxylierten Monoestern einer C₂-C₈Carbonsäure und eines C₂-C₈Alkohols;
- gegebenenfalls hydroxylierten Diestern einer C₂-C₈Dicarbonsäure und eines C₂-C₈Alkohols, wie z. B. Diisopropyladipat, 2-Diethylhexyladipat, Dibutyladipat oder Diisostearyladipat;
- gegebenenfalls hydroxylierten Triestern einer C₂-C₈Tricarbonsäure und eines C₂-C₈Alkohols, wie z. B. Citronensäureester, wie z. B. Trioctylcitrat, Triethylcitrat, Acetyltributylcitrat, Tributylcitrat oder Acetyltributylcitrat;
- Estern eines C₂-C₈Polyols und einer oder mehrerer C₂-C₈Carbonsäuren, wie z. B. Glycoldiester von Monosäuren, wie z. B. Neopentylglycoldiheptanoat, oder Glycoltriester von Monosäuren, wie z. B. Triacetin.

5. Zusammensetzung gemäß dem vorstehenden Anspruch, **dadurch gekennzeichnet, dass** die nichtflüchtigen Öl(e) auf Kohlenwasserstoffbasis (E) ausgewählt sind aus C₁₀-C₂₆Alkoholen, vorzugsweise Monoalkoholen, vorzugsweise ausgewählt aus Laurylalkohol, Isostearylalkohol, Oleylalkohol, Erucylalkohol, 2-Hexyldecylalkohol, Isocetylalkohol und Octyldodecanol und Gemischen davon, vorzugsweise Octyldodecanol.

6. Zusammensetzung gemäß einem der vorstehenden Ansprüche, **dadurch gekennzeichnet, dass** die nichtflüchtigen ersten Öl(e) auf Kohlenwasserstoffbasis (E) mit einem Gehalt in dem Bereich von 5 Gew.-% bis 75 Gew.-%, insbesondere von 10 Gew.-% bis 50 Gew.-% und vorzugsweise von 20 Gew.-% bis 45 Gew.-% bezogen auf das Gesamtgewicht der Zusammensetzung vorhanden sind.

7. Zusammensetzung gemäß einem der vorstehenden Ansprüche, **dadurch gekennzeichnet, dass** die nichtflüchtigen zweiten Öl(e) (F) ausgewählt sind aus Phenylsiliconölen, vorzugsweise ausgewählt aus Phenyltrimethiconen, Phenyldimethiconen, Phenyltrimethylsiloxydiphenylsiloxanen, Diphenyldimethiconen, Diphenylmethyldiphenyltrisiloxanen, 2-Phenylethyltrimethylsiloxysilicaten, Trimethylsiloxyphenyldimethiconen und Gemischen davon, vorzugsweise Trimethylsiloxyphenyldimethiconen.

8. Zusammensetzung gemäß einem der vorstehenden Ansprüche, **dadurch gekennzeichnet, dass** die nichtflüchtigen zweiten Öl(e) auf Kohlenwasserstoffbasis (F), die von den Ölen (E) verschieden sind, ausgewählt sind aus:
• Esterölen, die wenigstens 18 Kohlenstoffatome umfassen, insbesondere z. B.:
* Monoester, die wenigstens 18 Kohlenstoffatome umfassen,
* Monoester einer Fettsäure, die wenigstens 18 Kohlenstoffatome umfasst, und eines Diols,
* Diester, die wenigstens 18 Kohlenstoffatome umfassen,
* hydroxylierte Monoester und Diester, die wenigstens 18 Kohlenstoffatome umfassen,
* Triester, die wenigstens 35 Kohlenstoffatome umfassen,
* Tetraester, die wenigstens 35 Kohlenstoffatome umfassen,
* Polyester, erhalten durch Kondensation eines Dimers und/oder Trimers einer ungesättigten Fettsäure und eines Diols,
* Ester und Polyester eines Dioldimers und einer Monocarbonsäure oder Dicarbonsäure,
* Polyester, erhalten durch Verestern wenigstens eines Triglycerids von hydroxylierten Carbonsäure(n) mit einer aliphatischen Monocarbonsäure und mit einer aliphatischen Dicarbonsäure, die gegebenenfalls ungesättigt ist,
* Pflanzenöle auf Kohlenwasserstoffbasis,
* Saccharoseester,
• Ölen, die wenigstens eine Carbonatfunktion umfassen,
• Gemischen davon.

9. Zusammensetzung gemäß einem der vorstehenden Ansprüche, **dadurch gekennzeichnet, dass** sie einen Gehalt in dem Bereich von 5 Gew.-% bis 75 Gew.-% an nichtflüchtigen zweiten Öl(en) (F) umfasst, insbesondere von 5 Gew.-% bis 60 Gew.-% und besonders von 10 Gew.-% bis 50 Gew.-% bezogen auf das Gesamtgewicht der Zusammensetzung.

10. Zusammensetzung gemäß einem der vorstehenden Ansprüche, **dadurch gekennzeichnet, dass** sie von 5 Gew.-% bis 80 Gew.-%, vorzugsweise von 10 Gew.-% bis 70 Gew.-%, insbesondere von 15 Gew.-% bis 60 Gew.-% und noch besser von 20 Gew.-% bis 50 Gew.-% Wasser bezogen auf das Gesamtgewicht umfasst.

11. Zusammensetzung gemäß einem der vorstehenden Ansprüche, **dadurch gekennzeichnet, dass** das hydrophile Geliermittel ausgewählt ist aus:
- Galactomannanen und Derivaten davon, wie z. B. Johannisbrotkernmehl, Bockshornkleemehl, Cassia-Gummi und Guargummi und Derivate davon;
- Biopolysaccharidgummis mit mikrobiellem Ursprung, wie z. B. Skleroglucangummi oder Xanthangummi;
- Gummis, die aus Pflanzenexsudaten abgeleitet sind, wie z. B. Gummi arabicum, Ghatti-Gummi, Karaya-Gummi, Tragacanth-Gummi, Carrageenan-Gummi, Agar-Gummi und Johannisbrotkernmehl;
- Pectinen, Alginaten, Stärken;
- Gemischen davon.

12. Zusammensetzung gemäß einem der vorstehenden Ansprüche, **dadurch gekennzeichnet, dass** das hydrophile Geliermittel ausgewählt ist aus Guargummis und Derivaten davon, vorzugsweise ausgewählt aus Guargummi, Hydroxypropyl-Guar, mit Natriummethylcarboxylatgruppen modifiziertem Hydroxypropyl-Guar, Hydroxypropyltrimethylammonium-Guar-Chlorid, Xanthanderivaten und Gemischen davon, vorzugsweise Hydroxypropyl-Guar.

13. Zusammensetzung gemäß einem der vorstehenden Ansprüche, **dadurch gekennzeichnet, dass** der Gehalt an hydrophilem Geliermittel von 0,1 Gew.-% bis 20 Gew.-%, vorzugsweise von 0,1 Gew.-% bis 10 Gew.-% und insbesondere von 0,1 Gew.-% bis 5 Gew.-% bezogen auf das Gewicht der Zusammensetzung darstellt.

14. Zusammensetzung gemäß einem der vorstehenden Ansprüche, **dadurch gekennzeichnet, dass** der Gehalt an Polyol von 0,1 Gew.-% bis 20 Gew.-%, vorzugsweise von 1 Gew.-% bis 10 Gew.-% und insbesondere von 2 Gew.-% bis 6 Gew.-% bezogen auf das Gewicht der Zusammensetzung darstellt.

15. Zusammensetzung gemäß einem der vorstehenden Ansprüche, **dadurch gekennzeichnet, dass** sie wenigstens ein anionisches grenzflächenaktives Mittel umfasst, vorzugsweise ausgewählt aus Alkalimetallalkylsulfaten und -alkylethersulfaten, die wenigstens als 12 Kohlenstoffatome enthalten, insbesondere von 12 bis 16 Kohlenstoffatome, und gegebenenfalls von 1 bis 20 Oxyethyleneinheiten umfassen, und Gemischen davon.

16. Zusammensetzung gemäß dem vorstehenden Anspruch, **dadurch gekennzeichnet, dass** der Gehalt an anionischem grenzflächenaktivem Mittel von 0,1 Gew.-% bis 2 Gew.-%, vorzugsweise von 0,2 Gew.-% bis 1,5 Gew.-%, bezogen auf das Gewicht der Zusammensetzung darstellt.

17. Zusammensetzung gemäß einem der vorstehenden Ansprüche, **dadurch gekennzeichnet, dass** sie wenigstens ein nichtionisches grenzflächenaktives Mittel umfasst, vorzugsweise ausgewählt aus Alkyl- und Polyalkylestern von Sorbitan, die vorzugsweise nicht polyoxyethyleniert sind; Fettalkoholen, die wenigstens 8 Kohlenstoffatome enthalten und gegebenenfalls von 1 bis 150 Oxyethyleneinheiten und/oder Oxypropyleneinheiten umfassen; und Gemischen davon.

18. Zusammensetzung gemäß dem vorstehenden Anspruch, **dadurch gekennzeichnet, dass** der Gehalt an nichtionischen grenzflächenaktiven Mittel(n) von 0,5 Gew.-% bis 10 Gew.-%, vorzugsweise von 1 Gew.-% bis 5 Gew.-%, bezogen auf das Gewicht der Zusammensetzung darstellt.

19. Zusammensetzung gemäß einem der vorstehenden Ansprüche, **dadurch gekennzeichnet, dass** sie wenigstens ein Geminitensid der Formel (I) umfasst: 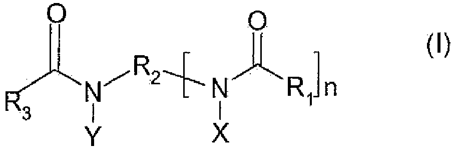 wobei:
* R₁ und R₃ unabhängig voneinander einen Alkylrest darstellen, der von 1 bis 25 Kohlenstoffatome enthält;
* R₂ eine Abstandsgruppe darstellt, die aus einer linearen oder verzweigten Alkylenkette, die von 1 bis 12 Kohlenstoffatome enthält, besteht;
* X und Y unabhängig voneinander eine Gruppe -(C₂H₄O)ₐ-(C₃H₆O)_{b}Z darstellen, wobei
* Z ein Wasserstoffatom oder einen Rest -CH₂-COOM, -SO₃M, -P(O)(OM)₂, -C₂H₄-SO₃M, -C₃H₆-SO₃M oder -CH₂(CHOH)₄CH₂OH darstellt, wobei M und M' H oder ein Alkalimetall- oder Erdalkalimetall- oder Ammonium- oder Alkanolammoniumion darstellen,
* a in dem Bereich von 0 bis 15 liegt, b in dem Bereich von 0 bis 10 liegt und die Summe von a + b in dem Bereich von 1 bis 25 liegt; und
* n in dem Bereich von 1 bis 10 liegt.

20. Zusammensetzung gemäß dem vorstehenden Anspruch, **dadurch gekennzeichnet, dass** sie ein Gemisch von 10 Gew.-% bis 20 Gew.-% Natriumdicocoylethylendiamin-PEG-15-Sulfat, von 30 Gew.-% bis 40 Gew.-% Glycerylstearat, von 10 Gew.-% bis 20 Gew.-% Glycerylstearatmonocitrat und von 30 Gew.-% bis 40 Gew.-% Behenylalkohol bezogen auf das Gesamtgewicht des Gemischs von grenzflächenaktiven Mitteln, das das Geminitensid umfasst, umfasst.

21. Zusammensetzung gemäß einem der Ansprüche 19 und 20, **dadurch gekennzeichnet, dass** das Geminitensid der Formel (I) mit einem Gehalt in dem Bereich von 1 Gew.-% bis 3 Gew.-% bezogen auf das Gesamtgewicht der Zusammensetzung vorhanden ist, vorzugsweise in dem Bereich von 0,2 Gew.-% bis 2 Gew.-% bezogen auf das Gesamtgewicht der Zusammensetzung.

22. Zusammensetzung gemäß einem der vorstehenden Ansprüche, **dadurch gekennzeichnet, dass** sie wenigstens einen Farbstoff umfasst.

23. Zusammensetzung gemäß einem der vorstehenden Ansprüche, **dadurch gekennzeichnet, dass** die Zusammensetzung eine Zusammensetzung zum Make-up und/oder zur Pflege von Keratinmaterialien, insbesondere der Lippen, ist.

24. Kosmetisches Verfahren zum Make-up und/oder zur Pflege von Keratinmaterialien, insbesondere der Lippen, umfassend wenigstens einen Schritt, der aus dem Aufbringen wenigstens einer Zusammensetzung gemäß einem der vorstehenden Ansprüche auf die Keratinmaterialien besteht.

## Revendications

1. Composition cosmétique sous la forme d'une émulsion huile dans eau comprenant :
(A) au moins 5 % en poids d'eau par rapport au poids total de la composition ;
(B) au moins de 4 % en poids d'alkylcellulose, dont le résidu alkyle comprend entre 2 et 6 atomes de carbone ;
(C) au moins un gélifiant hydrophile choisi parmi les polymères d'origine naturelle, éventuellement modifiés ;
(D) au moins un polyol en C₂-C₈, de préférence en C₃-C₆, saturé ou non, linéaire ou ramifié, comprenant de 2 à 6 groupements hydroxyle,
(E) au moins une première huile non volatile hydrocarbonée choisie parmi :
- les alcools en C₁₀-C₂₆, de préférence les monoalcools ;
- les monoesters, les diesters, les triesters, optionnellement hydroxylés, d'un acide mono ou polycarboxylique en C₂-C₈ et d'un alcool en C₂-C₈, optionnellement hydroxylés ;
- les esters d'un polyol en C₂-C₈ et d'un ou plusieurs acides carboxyliques en C₂-C₈ ; et
(F) au moins une seconde huile non volatile choisie parmi les huiles siliconées et/ou fluorées ou les huiles hydrocarbonées différentes de ladite première huile.

2. Composition selon la revendication précédente, **caractérisée en ce que** l'alkylcellulose est présente en une teneur variant de 4 % à 60 % en poids, de préférence de 4 % à 50 % en poids, mieux de 5 % à 30 % en poids, et plus préférentiellement de 5 à 20 % en poids, par rapport au poids total de la composition.

3. Composition selon l'une quelconque des revendications précédentes, **caractérisée en ce que** l'alkylcellulose est choisie parmi l'éthylcellulose et la propylcellulose, de préférence est l'éthylcellulose.

4. Composition selon l'une quelconque des revendications précédentes, **caractérisée en ce que** la ou les premières huiles non volatiles hydrocarbonées (E) sont choisies parmi :
- l'alcool laurique, l'alcool stéarylique, l'alcool isostéarylique, l'alcool oléique, l'alcool érucique, l'alcool 2-hexyldécylique, l'alcool isocétylique, l'octyldodécanol et leurs mélanges ;
- les monoesters optionnellement hydroxylés, d'un acide carboxylique en C₂-C₈ et d'un alcool en C₂-C₈ ;
- les diester optionnellement hydroxylés, d'un diacide carboxylique en C₂-C₈ et d'un alcool en C₂-C₈, tels que le diisopropyl adipate, le diéthyl-2 hexyl adipate, le dibutyl adipate, ou le diisostéaryle adipate;
- les triesters optionnellement hydroxylés d'un triacide carboxylique en C₂-C₈ et d'un alcool en C₂-C₈, tels que les esters d'acide citrique, tels que le trioctyle citrate, triéthylcitrate, l'acétyltributyl citrate, le tributyl citrate, l'acétyltributyl citrate ;
- les esters d'un polyol en C₂-C₈ et d'un ou plusieurs acides carboxyliques en C₂-C₈, tels que les diesters de glycol et de monoacides, tels que le diheptanoate de néopentylglycol, ou les triesters de glycol et de monoacides tel que la triacétine.

5. Composition selon la revendication précédente, **caractérisée en ce que** la ou les huiles non volatiles hydrocarbonées (E), sont choisies parmi les alcools en C₁₀-C₂₆, de préférence les monoalcools, de préférence choisies parmi l'alcool laurique, l'alcool isostéarylique, l'alcool oléique, l'alcool érucique, l'alcool 2-hexyldécylique, l'alcool isocétylique, l'octyldodécanol et leurs mélanges, de préférence l'octyldodécanol.

6. Composition selon l'une quelconque des revendications précédentes, **caractérisée en ce que** la ou les premières huiles hydrocarbonées non volatiles (E) sont présentes en une teneur allant de 5 % à 75 %, en particulier de 10 % à 50 % en poids, de préférence de 20 % à 45% en poids, par rapport au poids total de la composition.

7. Composition selon l'une quelconque des revendications précédentes, **caractérisée en ce que** la ou les deuxièmes huiles non volatiles (F) sont choisies parmi les huiles siliconées phénylées, de préférence choisies parmi les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates, les triméthylsiloxyphényldiméthicones, et leurs mélanges, et de préférence les triméthylsiloxyphényldiméthicones.

8. Composition selon l'une quelconque des revendications précédentes, **caractérisée en ce que** la ou les secondes huiles non volatiles hydrocarbonées (F), différentes des huiles (E) sont choisies parmi :
• les huiles ester ayant au moins 18 atomes de carbone, comme notamment :
* les monoesters comprenant au moins 18 atomes de carbone
* les monoesters d'acide gras comprenant au moins 18 atomes de carbone et de diol
* les diesters comprenant au moins 18 atomes de carbone
* les monoesters et les diesters hydroxylés comprenant au moins 18 atomes de carbone
* les triesters comprenant au moins 35 atomes de carbone
* les tétraesters comprenant au moins 35 atomes de carbone
* les polyesters obtenus par condensation de dimère et/ou trimère d'acide gras insaturé et de diol
* les esters et polyesters de dimère diol et d'acide mono- ou dicarboxylique
* les polyesters résultant de l'estérification d'au moins un triglycéride d'acide(s) carboxylique(s) hydroxylé(s) par un acide monocarboxylique aliphatique et par un acide dicarboxylique aliphatique, éventuellement insaturé
* les huiles hydrocarbonées végétales
* les esters de sucrose
• les huiles comprenant au moins une fonction carbonate
• leurs mélanges.

9. Composition selon l'une quelconque des revendications précédentes, **caractérisée en ce qu'**elle comprend une teneur allant de 5 % à 75 % en poids de deuxième(s) huile(s) non volatile(s) (F), en particulier de 5 % à 60 % en poids, et plus particulièrement de 10 % à 50 % en poids, par rapport au poids total de la composition.

10. Composition selon l'une quelconque des revendications précédentes, **caractérisée en ce qu'**elle comprend de 5 % à 80 % en poids d'eau, de préférence de 10 % à 70 % en poids, en particulier de 15 % à 60 % en poids, et mieux de 20 % à 50 %, en poids, par rapport à son poids total.

11. Composition selon l'une quelconque des revendications précédentes, **caractérisée en ce que** le gélifiant hydrophile est choisi parmi :
- les galactomannanes et leurs dérivés, tels que la gomme de Caroube, la gomme de Fennugrec, la gomme de cassia, les gommes de guar et leurs dérivés ;
- les gommes de biopolysaccharides d'origine microbienne telles que les gommes de Scléroglucane ou de Xanthane ;
- les gommes issues d'exudats végétaux telles que les gommes Arabique, Ghatti, Karaya, Tragacanthe, Carraghénane, Agar et Caroube;
- les pectines, les alginates, les amidons ;
- leurs mélanges.

12. Composition selon l'une quelconque des revendications précédentes, **caractérisée en ce que** le gélifiant hydrophile est choisi parmi les gommes de guar et leurs dérivés, de préférence choisi parmi la gomme de guar, l'hydroxypropylguar, l'hydroxypropylguar modifié par des groupements méthylcarboxylate de sodium, le chlorure de guar hydroxypropyl tri-méthyl ammonium, les dérivés du xanthane, et leurs mélanges ; de préférence l'hydroxypropyl guar.

13. Composition selon l'une quelconque des revendications précédentes, **caractérisée en ce que** la teneur en gélifiant hydrophile représente de 0,1 % à 20 % en poids, de préférence de 0,1 % à 10 % en poids, et plus particulièrement de 0,1 % à 5 % en poids, par rapport au poids de la composition.

14. Composition selon l'une quelconque des revendications précédentes, **caractérisée en ce que** la teneur en polyol représente de 0, 1 à 20 % en poids, de préférence de 1 à 10 % en poids, et plus particulièrement de 2 à 6 % en poids, par rapport au poids de la composition.

15. Composition selon l'une quelconque des revendications précédentes, **caractérisée en ce qu'**elle comprend au moins un tensioactif anionique, de préférence choisi parmi les alkylsulfate et alkyléthersulfate de métaux alcalins, ayant au moins 12 atomes de carbone, plus particulièrement de 12 à 16 atomes de carbone, et comprenant éventuellement de 1 à 20 motifs oxyéthylénés ainsi que leurs mélanges.

16. Composition selon la revendication précédente **caractérisée en ce que** la teneur en tensioactif anionique représente de 0,1 % à 2 % en poids, de préférence de 0,2 % à 1,5 % en poids, par rapport au poids de la composition.

17. Composition selon l'une quelconque des revendications précédentes, **caractérisée en ce qu'**elle comprend au moins un tensioactif non ionique, de préférence choisi parmi les alkyl- et polyalkyl- esters de sorbitan, de préférence non polyoxyéthylénés ; les alcools gras, ayant au moins 8 atomes de carbone, et pouvant comporter de 1 à 150 motifs oxyéthylène et/ou oxypropylène ; leurs mélanges.

18. Composition selon la revendication précédente, **caractérisée en ce que** la teneur en tensioactif(s) non ionique(s) représente de 0,5 % à 10 % en poids, de préférence de 1 % à 5 % en poids, par rapport au poids de la composition.

19. Composition selon l'une quelconque des revendications précédentes, **caractérisée en ce qu'**elle comprend au moins un agent tensioactif géminé de formule (I) : 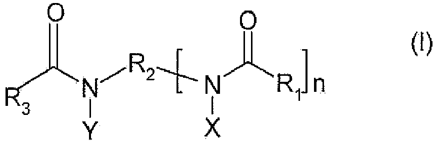 dans laquelle :
* R₁ et R₃ désignent, indépendamment l'un de l'autre, un radical alkyle ayant de 1 à 25 atomes de carbone ;
* R₂ désigne un groupement espaceur constitué d'une chaîne alkylène linéaire ou ramifiée ayant de 1 à 12 atomes de carbone ;
* X et Y désignent, indépendamment l'un de l'autre, un groupement
-(C₂H₄O)ₐ-(C₃H₆O)_{b}Z
où
* Z désigne un atome d'hydrogène ou un radical -CH₂-COOM, -SO₃M, -P(O)(OM)₂, -C₂H₄-SO₃M, -C₃H₆-SO₃M ou -CH₂(CHOH)₄CH₂OH, où M, M' représentent H ou un ion de métal alcalin ou de métal alcalino-terreux ou ammonium ou alcanolammonium,
* a va de 0 à 15, b va de 0 à 10, et la somme de a + b va de 1 à 25 ; et
* n va de 1 à 10.

20. Composition selon la revendication précédente, **caractérisée en ce qu'**elle comprend un mélange de 10 % à 20 % en poids de Sodium Dicocoyléthylènediamine PEG-15 Sulfate, de 30 % à 40 % en poids de stéarate de glycéryle, de 10 % à 20 % en poids de stéarate de monocitrate de glycéryle, de 30 % à 40 % en poids d'alcool béhénylique, par rapport au poids total du mélange de tensioactifs contenant le tensioactif géminé.

21. Composition selon l'une quelconque des revendications 19 et 20 **caractérisée en ce que** le tensioactif géminé de formule (I) est présent en une teneur allant de 1 % à 3 % en poids, par rapport au poids total de la composition, de préférence allant de 0,2 % à 2 % en poids par rapport au poids de la composition.

22. Composition selon l'une quelconque des revendications précédentes, **caractérisée en ce qu'**elle comprend au moins une matière colorante.

23. Composition selon l'une quelconque des revendications précédentes, **caractérisée en ce que** ladite composition est une composition de maquillage et/ou de soin des matières kératiniques, en particulier des lèvres,

24. Procédé cosmétique de maquillage et/ou de soin des matières kératiniques, en particulier des lèvres, comprenant au moins une étape consistant à appliquer sur lesdites matières kératiniques au moins une composition telle que définie selon l'une quelconque des revendications précédentes.
